(19) Europäisches Patentamt / European Patent Office / Office européen des brevets

(11) **EP 3 939 514 B1**

## (12) EUROPEAN PATENT SPECIFICATION

(45) Date of publication and mention
of the grant of the patent:
**26.03.2025 Bulletin 2025/13**

(21) Application number: **20305804.5**

(22) Date of filing: **15.07.2020**

(51) International Patent Classification (IPC):
$A61B\ 8/08^{(2006.01)}$    $G01N\ 33/49^{(2006.01)}$
$G16H\ 50/30^{(2018.01)}$    $G16H\ 50/20^{(2018.01)}$
$A61B\ 5/151^{(2006.01)}$    $A61B\ 5/157^{(2006.01)}$
$A61B\ 5/15^{(2006.01)}$

(52) Cooperative Patent Classification (CPC):
**A61B 8/485; A61B 5/0051; A61B 5/150022;
A61B 5/150358; A61B 5/150854; A61B 5/150862;
A61B 5/15087; A61B 5/157; A61B 5/4244;
A61B 8/5223; G01N 33/49; G16H 50/20;
G16H 50/30**

(54) **SYSTEM AND METHOD FOR DETERMINING A HEALTH CONDITION OF THE LIVER OF A SUBJECT**

SYSTEM UND VERFAHREN ZUR BESTIMMUNG DES GESUNDHEITSZUSTANDS DER LEBER EINES PATIENTEN

SYSTÈME ET PROCÉDÉ PERMETTANT DE DÉTERMINER UN ÉTAT DE SANTÉ DU FOIE D'UN SUJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.01.2022 Bulletin 2022/03**

(73) Proprietor: **Echosens**
**75014 Paris (FR)**

(72) Inventors:
- **SANDRIN, Laurent**
**75014 PARIS (FR)**
- **MIETTE, Véronique**
**75014 PARIS (FR)**
- **DESTRO, Marie**
**75014 PARIS (FR)**
- **KAHATT, Espir**
**CARLSBAD, CA 92011 (US)**
- **WILLIAMS, Michael**
**CARLSBAD, CA 92011 (US)**
- **KOSTELESKI, Adrian**
**MOORESTOWN, NJ 08057 (US)**

(74) Representative: **Cabinet Camus Lebkiri**
**25 rue de Maubeuge**
**75009 Paris (FR)**

(56) References cited:
**EP-A1- 2 600 266**    **WO-A2-2015/014763**
**KR-A- 20180 003 061**    **US-A1- 2018 140 247**

- **DA BEN L ET AL: "The Delta-4 fibrosis score
(D4FS): A novel fibrosis score in chronic
hepatitis D", ANTIVIRAL RESEARCH, ELSEVIER
BV, NL, vol. 174, 16 December 2019 (2019-12-16),
XP086051223, ISSN: 0166-3542, [retrieved on
20191216], DOI: 10.1016/
J.ANTIVIRAL.2019.104691**
- **FRAQUELLI M ET AL: "A SEQUENTIAL
PREDICTIVE ALGORITHM COMBINING APRI
AND TRANSIENT ELASTOGRAPHY (TE) FOR
DIAGNOSING LIVER FIBROSIS IN CHRONIC
HEPATITIS C", DIGESTIVE AND LIVER DISEASE,
W.B. SAUNDERS, GB, vol. 41, 1 March 2009
(2009-03-01), pages S31, XP026191117, ISSN:
1590-8658, [retrieved on 20090301], DOI: 10.1016/
S1590-8658(09)60079-7**

**Description**

TECHNICAL FIELD

The disclosed technology concerns a system and a method for determining a health condition of the liver of a subject, in particular on the basis of both a liver stiffness measurement and a liver enzyme concentration in the subject's blood.

BACKGROUND

Liver stiffness measured by Vibration-Controlled Transient Elastography has been shown to be an efficient tool to diagnose chronic liver diseases like fibrosis. The article "Transient elastography: a new noninvasive method for assessment of hepatic fibrosis", Ultrasound in Medicine and Biology, Volume 29, Number 12, 2003, by L. Sandrin et al., for instance, shows that liver stiffness correlates well with liver fibrosis. But liver stiffness in influenced by other factors such as inflammation and congestion.

Interestingly, liver inflammation can be assessed by measuring the concentration of one or more liver enzymes in blood (as liver inflammation usually leads to high levels of these enzymes). So, knowing the concentration of one or more liver enzymes in blood allows for a better interpretation of measured values of liver stiffness and enables to obtain reliable information regarding both a possible inflammation/congestion and a possible fibrosis/cirrhosis, as shown in the article "Liver stiffness: a novel parameter for the diagnosis of liver disease", Hepatic Medicine: Evidence and Research, vol. 2, pp 49-67, 2010, by S. Muller and L. Sandrin.

To take into account and gather these two parameters (serologic, and mechanical), when determining the health condition of the liver of subject, one may compute the value of a benchmark parameter that depends both on liver stiffness and on the concentration(s) of liver enzyme(s), and that is specifically designed to allow for the identification of different health conditions (or disease conditions) of the liver of the subject. Such a benchmark parameter is sometimes designated as a "score", in medical publications. The article "FibroScan-AST (FAST) score for the non-invasive identification of patients with non-alcoholic steatohepatitis with significant activity and fibrosis: a prospective derivation and global validation study", The Lancet, Gastroenterology and Hepatology, Volume 5, issue 4, pp 362-373, April 01, 2020, by P. Newsome et al., for instance, presents such a benchmark parameter, called "FAST", that takes into account the liver stiffness "LSM", an ultrasound attenuation parameter in liver referred to as "CAP" (Controlled Attenuation Parameter), and a concentration of aspartate aminotransferase (AST) in blood. This benchmark parameter is computed according to formula F1 below (where AST is expressed in IU/L):

$$FAST = \frac{exp(-1.65 + 1.07 \times ln[LSM] + 2.66 \times 10^{-8} \times CAP^3 - 63.3/AST)}{1 + exp(-1.65 + 1.07 \times ln[LSM] + 2.66 \times 10^{-8} \times CAP^3 - 63.3/AST)} \quad (F1)$$

Anyhow, to detect and characterize a possible liver disease in a subject by taking into account both liver stiffness and the concentration of one or more liver enzymes, one has first to measure these two quantities.

Liver stiffness is typically measured by Vibration-Controlled Transient Elastography. Such a measurement is completely non-invasive and is typically carried on in a medical imaging lab environment. And to determine the concentration of one or more liver enzymes in the subject's blood, a health care professional, trained to collect biological samples, collects a sample of venous blood by means of a syringe, and sends this sample to a bio-medical analysis laboratory where this blood sample is analyzed. Once the values of liver stiffness and liver enzyme(s) concentration(s) are both available, the health condition of the liver of the subject can be assessed, for instance by computing a benchmark parameter as the one presented above.

But this way to proceed has several drawbacks.

First, the requirements, in terms of examination staff and environment, are rather different for such a serologic analysis based on venous blood, and for a liver stiffness measurement. So, these two examinations are generally carried on in different places or environments (and so at different moments), which complicates the overall procedure, makes it more expensive, and increases the possibility that an error occurs when transferring the examination partial results.

Besides, in this procedure, the blood analysis results are not available immediately, and a complete diagnosis can only be achieved a posteriori. So, when the combined examination results (both physical and serologic) finally indicate a possibly liver disease, corresponding for instance to an unusually high value of the FAST parameter, the health care professional is no longer in a position to repeat one, or both of these examinations in order to confirm the diagnosis, which may increase the rate of false positive detection.

And this kind of independence of the blood analysis with respect to the stiffness and/or ultrasound attenuation measurement leads to a systematic analysis of the subject's blood whereas such a blood test turns out to be useless in some situations. For instance, given that the negative predictive value (NPV) of liver stiffness is excellent, a very low liver stiffness, typically smaller than 6 to 7 kPa, indicates that the subject under examination is not likely to suffer from any fibrosis, regardless of the concentrations of liver enzymes in the subject's blood (as explained in the article by Muller and Sandrin cited above). So, in such a situation, testing the subject's blood, as it is usually done, is a useless expenditure of resources.

Collecting venous blood enables to have quite a large amount of blood at disposal, thus permitting a fine and accurate blood analysis. But in return, it increases the delay (and possible travel time) between the blood sample collection and its analysis. And it turns out that this delay varies significantly from one hospital or medical testing center to another, and even from day to day in the same hospital or medical testing center. And the chemical activity of liver enzymes in a blood sample significantly decreases with time once the sample has been collected. So, for a given blood sample, the concentration of a given liver enzyme that is actually measured (in other words, the apparent concentration of this enzyme) varies strongly with the delay between collection and analysis. So, the variability of the collection-to-analysis delay leads to a variability of the liver enzymes results themselves, which is one of the reasons of the indication of normal values along with the measured values.

Besides, in this measurement process, the blood sample collection and the liver stiffness measurement can be carried on at very different moments. This absence of coordination too may affect the repeatability of the final diagnosis, as the concentration of liver enzymes in a subject's blood varies over time, due to circadian and other variations.

In this context, one knows blood-test devices called "Point of Care" devices (or "POC") that enable to analyze a blood sample in-situ, with no need to send and carry this sample to a central laboratory for testing. Some of these POC devices, like the Piccolo Xpress model by Abaxis (Piccolo Xpress is a registered trademark) provide results accurate enough for further benchmark and score computations. With this device, to test the blood of a subject, a blood sample of approximately 100 microliters is collected from the subject and is then injected in a disk-like disposable using a micropipette. This disposable is then inserted in a blood-test reader where the disposable is rotated to centrifugate the blood sample in order to separate the blood's cellular components from other blood components. The sample then reacts with reagents, contained in the disposable and appropriate to detect optically various blood components such as liver enzymes. Employing this POC device, instead of sending a blood sample to a central laboratory for testing, could improve the process presented above. But this POC device requires a rather large volume of blood (~0.1 mL). So, the issues with the collection of such a blood sample are quite the same as for the collection of venous blood, and this collection often requires the intervention of a health care professional (all the more that the injection of the collected blood sample into the disposable is rather difficult).

And anyhow, even if the concentration of liver enzyme in the subject's blood would be carried on with this POC device, the serological measurement and the stiffness measurement would remain decoupled from each other, and the result of this process would therefore remain subject to the variations and fluctuations mentioned above.

It would therefore be desirable to further improve such a process for determining a health condition of the liver of a subject, based on both a serological and a stiffness measurement. In particular, it would be desirable to further improve its reproducibility, to allow for a better control of the examination conditions and process, to simplify it, and to reduce the associated costs.

Document US 2018/140247 describes a method for determining a score related to the condition of the liver of the subject, based on an elastography measurement and a measurement of the activity of ASAT/ALAT/GGT, for the subject considered.

Document WO 2015/014763 describes a method for calculating a human or animal score, comprising an elastography measurement and a measurement of biological parameters thanks to a Point Of Care device.

SUMMARY

The invention is defined in the appended claims.

To resolve at least partially the problems mentioned above, the disclosed technology is a system and a method for determining a health condition of the liver of a subject:

- in which a blood-test reader is operatively connected to an elastography device, allowing for a coordinated, for instance concomitant operation of these devices, and
- in which a blood-test disposable associated to the blood-test reader is configured to enable a convenient and easy collection of a capillary blood sample, even for a non-professional individual.

More specifically, the disclosed technology concerns a system for determining a health condition of the liver of a subject, the system comprising:

- an elastography device configured to measure at least one mechanical parameter relative to the propagation of shear waves in liver,

- a blood-test reader and a blood-test disposable associated to the blood test reader, the blood-test disposable being configured to receive a capillary blood sample and to be inserted in the blood test reader and the blood-test reader being configured to determine a concentration of at least one liver enzyme in the blood sample, wherein:

- the blood-test disposable comprises:

    - a capillary tube for collecting the blood sample from the finger, and

    - reagents, appropriate to detect the at least one liver enzyme in the blood sample, and wherein

- the blood-test reader is operatively connected to the elastography device, and

- a control and processing system, comprising at least a processor and a memory, configured to control the elastography device and the blood test reader, the control and processing system being programmed to execute the following steps:

    - S1 - acquiring a value of the at least one mechanical parameter, measured by the elastography device,

    - S2 - acquiring a value of a concentration of the at least one liver enzyme in a blood sample collected from the subject, measured by the blood-test reader, and

    - S3 - determining a health condition of the liver of the subject, taking into account both the value of the at least one mechanical parameter and the value of the concentration of the at least one liver enzyme, the health condition being represented by a health or disease stage identified among different stages of a given health condition classification, or being represented by a value of a benchmark parameter taking into account both the value of the at least one mechanical parameter and the value of the concentration of the at least one liver enzyme, or being represented both by the health or disease stage and by the value of the benchmark parameter, and outputting data representative of the health condition.

Collecting a blood sample, in particular a small volume of capillary blood sample, by means of a capillary tube can be achieved easily and even by an individual who is not a health care professional trained to collect biological samples. Besides, as the capillary tube is integrated to the disposable, the delicate injection of a collected blood sample into the prior art disk-like disposable presented above, is avoided.

Thanks to the particular structure of the blood-test disposable presented above, a health care professional specifically trained to collect biological samples, and an environment appropriate for biological samples collection are no more required. Thanks to this particular disposable (and associated reader), the overall examination procedure can thus be achieved by a single operator, such as a health care professional trained for medical imaging examination (instead of two different health care professionals, having different specialties), or even by the subject under examination himself, and within a single examination environment (with less stringent sanitary constraints than an environment for conventional biological sample collections). This simplifies the overall procedure and, more importantly, it helps greatly to achieve concomitant measurements of the liver stiffness and of a liver enzyme level, which improves significantly the reproducibility and reliability of the procedure, as explained in detail above.

The applicant underlines that collecting a small amount of capillary blood to measure the concentration of a liver enzyme, in view of determining a health condition of the liver of the subject, in particular by computing a benchmark parameter, is a rather unusual approach.

Indeed, determining a health condition of the subject's liver, and computing benchmark parameters require an accurate measurement of such a concentration, which is made difficult by the rather low concentrations of such enzymes in blood. This is why venous blood samples, which allow for extensive blood analysis, are usually collected and employed for such a determination.

But it turns out surprisingly that a liver enzyme concentration can be determined rather accurately from a capillary blood sample of limited volume and the accuracy of the results thus obtained is in fact appropriate for such a liver diagnosis. Indeed, using such a collection method enables to launch the blood analysis immediately after collection, thus avoiding the liver enzymes chemical activity decrease that takes place between collection and analysis (apparent concentration decrease), in the usual venous-blood analysis method. So, with a capillary blood sample, the relative accuracy of the measurement may be smaller than with a venous blood sample, but the enzyme activity is higher, which leads finally to a measurement accuracy appropriate for determining a health condition of a subject's liver.

One will appreciate that the expression "liver enzyme concentration" may designate an apparent concentration of this enzyme, as determined using a given detection reaction or set of reactions, or a chemical activity of the liver enzyme in question. In this document, the expressions "enzyme concentration", "enzyme chemical activity" or "enzyme activity" will be used indifferently.

To allow for an accurate determination of one or more liver enzyme activity from a small quantity of capillary blood, the inventors have developed a specifically designed blood-test disposable and reader. In particular, in order to reduce the volume of blood necessary to carry on this analysis, a specific blood-test disposable configured to allow for the determination of liver enzyme activity only was developed (instead of using general purpose blood-test disposable, configured to detect many different kinds of enzymes or blood components). This specific blood-test disposable may in particular be configured to allow for the determination of AST and/or ALT activity only.

Besides, as mentioned above, the fact that the blood-test reader and the elastography device are operatively connected to each other allows for a coordinated, in particular a concomitant operation of these two devices. And achieving the elastography measurement and the corresponding blood test at the same time, or within a given, limited time frame improves significantly the repeatability and reliability of diagnosis or other result finally obtained, as explained in detail above.

Thanks to this connection, the control and processing system may, in particular, control the elastography device and the blood test reader so that they start respectively an elastography measurement process, and a blood test process including collection and analysis of the blood sample, within a single time frame, which contributes to obtaining concomitant measurements (that is simultaneous or almost simultaneous measurements). The control and processing system may in particular be programmed so that the time frame has a duration of 30 minutes at most.

In an embodiment, the elastography device may for instance be configured to prompt an operator to achieve an elastography measurement of thethe mechanical parameter, when starting the measurement process in question. And the blood-test reader may also be configured to prompt the operator to collect a capillary blood sample from the subject, when starting the measurement process triggered by the control and processing system. This prompting could be transmitted to the operator in various manner, for instance by displaying information on a screen, by turning on an indicator light, or by opening a blood-test disposable dispenser.

In one aspect of the present invention, the control and processing system is programmed to transmit information prompting the operator to both collect a capillary blood sample from the subject and to achieve an elastography measurement within a given time, when the measurement processes in question start (such a prompting could be achieved by displaying a timer or a time bar, for example).

The control and processing system may also be programmed to check, once the elastography and liver enzyme measurements have been completed, that they were concomitant. To this end, the control unit may be programmed to test whether a time gap, between a measurement moment of the at least one mechanical parameter and a measurement moment of the concentration of the at least one liver enzyme in the blood sample, passes a given duration threshold. And the control and processing system may be programmed to output an error message specifying that the health condition assessment of the liver of the subject failed, or specifying that the health condition assessment of the liver of the subject may not be reliable, when these two measurements are not concomitant.

Besides, as the blood-test reader and the elastography device are operatively connected to each other (instead of operating independently from another), it is possible, with this system, to implement diagnosis procedures and workflows in which the two devices interact with each other to adapt the procedure dynamically, depending on the measurement results.

For instance, in an alternative aspect of the present invention, the control and processing system is programmed to execute the following steps:

- S10 - controlling the elastography device so that the elastography device starts an elastography measurement process, then
- the step S1, then
- if the value of the at least one mechanical parameter acquired in step S1 fulfils a given criterion:

  - S3' - determining the health condition of the liver of the subject taking into account the value of the at least one mechanical parameter regardless of a concentration of the at least one liver enzyme in the subject's blood, and outputting data representative of the health condition, while

- if the value of the at least one mechanical parameter does not fulfil the criterion:

  - S20 - controlling an operator interface so that the interface transmits information specifying that a blood test is recommended for the liver health assessment of the subject and/or prompting the operator to collect a capillary blood sample from the subject and to launch the analysis of this blood sample, then

- the step S2, and then

- the step S3.

Thanks to these features, the blood test is carried on only when it is expected to significantly improve the determination of the health condition of the liver of the subject, thus saving time and resources.

In particular, the control and processing system could be programmed to control the blood-test reader so that it starts a blood test only if the value of the at least one mechanical parameter, acquired in step S1, indicates that the liver stiffness of the subject is above a given threshold, for instance above 5-6 kPa.

More generally, the control and processing system could be programmed to determine that the value of the at least one mechanical parameter does not fulfil the criterion (and that a blood test is thus recommended) when the value passes a threshold value, corresponding to a limit between values for which liver does not suffer from health impairment in average, and values for which liver may suffer from health impairment.

In a further alternative aspect of the present invention, the system for determining the health condition of the liver of the subject may be configured to start the liver enzyme(s) measurement process first, and then to start the elastography measurement process (instead of starting with the elastography measurement, to determine whether the liver enzymes measurement would be useful or not).

Even with a blood-test disposable and reader optimized to allow for a quick determination of the concentration of the liver enzyme, this determination is not instantaneous and takes typically a few minutes (this duration is necessary for the detection reagents to react with liver enzymes). It is thus beneficial to start the liver enzyme(s) measurement process as soon as possible, at first, and to use the time required for the blood sample analysis to carry on the elastography measurement. Indeed, proceeding in this order reduces the time required to achieve the overall examination procedure.

In this last embodiment, the control and processing system is programmed more particularly to execute the following steps:

- S20'- controlling an operator interface so that the interface transmits information prompting an operator to collect a capillary blood sample from the subject and to launch the analysis of this blood sample, then, once the blood-test disposable has been inserted in the blood-test reader,
- S10 - controlling the elastography device so that the elastography device starts an elastography measurement process, then
- the steps S1 and S2, and then,
- the step S3.

Besides, in some embodiments:

- different computation formulas are associated, in the memory of the control and processing system, to different ranges of values of the at least one mechanical parameter, each computation formula corresponding to a given benchmark parameter for liver health assessment and each computation formula enabling to compute the corresponding benchmark parameter from the at least one mechanical parameter and from the concentration of the at least one liver enzyme in the blood sample,

- the control and processing system is programmed to select one of these benchmark parameters, by comparing the value of the at least one mechanical parameter, previously measured on the subject's liver, to the ranges of values associated respectively to these different benchmark parameters, and

- the control and processing system is programmed to compute a value of the benchmark parameter previously selected, according to the formula associated to this benchmark parameter, in step S3.

Regarding now the blood-test disposable and associated blood-test reader, in an embodiment, the blood-test disposable is configured so that the blood sample, collected by means of the capillary tube, has a volume of 60 microliters at most, or even of 40 or 30 microliters at most.

The volume of capillary blood that can be collected from one finger prick is typically 10 to 20 microliters. So, the blood sample mentioned above can be collected by achieving a limited number of finger pricks, or even a single one. The inconvenience for the subject is thus limited (one avoids in particular to puncture all the subject's fingers). And another blood sample of this kind can be collected again, if it turns out that the liver enzyme measurement should be done again.

In some embodiments, the capillary tube is fixed, for instance irremovably fixed, to a part of the disposable.

In particular, the blood test disposable may comprise:

- a cartridge, containing the reagents, and

- a detachable plug,

the capillary tube being fixed to the detachable plug, or to the cartridge, the plug and the cartridge being configured so that:

- the plug can be detached from the cartridge and then re-plugged onto the cartridge, and so that
- when the plug is plugged onto the cartridge, the capillary tube is hosted inside the disposable and the blood sample collected by the capillary tube comes into contact with the reagents.

So, to collect the blood sample, the operator (who could be the subject under examination himself) punctures the skin of at least one finger of the subject, for instance with a lancet. The operator also detaches the plug from the cartridge and then collects the droplet (or droplets) of capillary blood thus obtained, with the capillary tube. The operator then re-plugs the plug onto the cartridge. The capillary tube remains thus hosted inside the disposable most of the time, which prevents possible contaminations of the capillary tube, or possible contaminations by the capillary tube once the blood sample has been collected. Besides, thanks to this structure, the collected blood sample is consistently and effortlessly mixed with reagents in a reproducible way, and the delicate blood-sample injection into a disposable of the prior art is avoided.

According to an optional feature of the system described above, the plug is configured so that plugging it onto the cartridge causes a pressure increase that pushes the blood sample, collected by means of the capillary tube, into the fluidic circuitry. Besides, the cartridge may comprise fluidic circuitry, arranged to bring at least a part of the blood sample into contact with the reagents. And the plug may be configured so that the capillary tube is in fluidic connection with this fluidic circuitry when the plug is plugged onto the cartridge.

In an embodiment of the system that has been presented above:

- the at least one liver enzyme is one of: aspartate aminotransferase, hereinafter "AST", alanine aminotransferase, hereinafter "ALT", gamma-glutamyl transferase, hereinafter "GGT",

- the reagents are appropriate to detect the at least one liver enzyme optically,

- the blood-test disposable is configured so that at least a part of the blood sample mixes with the reagents in a first reaction zone, and

- the blood-test reader comprises at least a light source and a light sensor for determining the concentration of the at least one liver enzyme in the blood sample, by means of a light reflectance and/or transmittance measurement at the first reaction zone.

The blood-test disposable may comprise a filtering membrane arranged to filter blood red cells out of the blood sample collected from the subject in order to obtain a plasma sample, and is configured to bring at least part of the plasma sample into contact with the reagents.

This way of filtering, by means of this membrane, may not be as complete as other filtration methods (such as centrifugation), and it produces a plasma sample, not a serum sample, but this filtration is efficient enough for the subsequent liver enzyme detection, and it is faster and causes a smaller volume loss than previous methods (this volume loss is typically smaller than 60% of the initial volume of the collected blood sample), which is beneficial as the volume of the capillary blood sample collected is small, here, and as an objective of the disclosed technology is to reduce the time required to determine the health condition of the liver of the subject.

In an embodiment of the system that has been presented above:

- the at least one liver enzyme is AST or ALT,

- the reagents comprise alpha-Ketoglutarate, and L-Aspartic Acid or L-alanine respectively, and

- the blood-test disposable comprises the following catalysts: pyruvate oxidase and peroxidase, and comprises an indicator, that becomes colored when reacting with a product of the set of reactions that occur when the at least one liver enzyme is mixed with the reagents.

In particular, the indicator may comprise MAOS Trinder reagent, and the blood-test disposable may comprise a liquid buffer and may be configured so that the buffer mixes with the reagents when the blood-test disposable is inserted into the blood-test reader, the buffer being an aqueous solution containing tris(hydroxyméthyl)aminomethane, herein after TRIS.

The concentration of TRIS in the buffer may in particular be from 0.05 to 1 mole/liter, or even from 0.1 to 0.5 mole/liter.

Employing this indicator and buffer allows for a fast and accurate determination of the concentration of the at least one liver enzyme in the collected blood sample. In practice, with this system, the measurement results are obtained typically 10 minutes or less (typically 5 minutes) after the blood sample collection, and the measurement accuracy is better than 20%, typically around 10% or even less.

MAOS Trinder reagent designate N-Ethyl-N-(2-hydroxy-3-sulfopropyl)-3,5-dimethylaniline sodium salt. And tris(hydroxyméthyl)aminomethane (TRIS) designate 2-amino-2-hydroxymethylpropane-1,3-diol.

In an embodiment, the reagents mentioned above are contained in a dry detection pad, while the catalyst and indicator are contained in a dry detection substrate distinct from the pad, the substrate and pad being arranged, in the first reaction zone mentioned above, so that at least a part of the plasma sample, possibly mixed with the buffer, soaks the detection pad and the substrate pad, thus allowing for the liver enzyme detection.

The liquid buffer mentioned above may also contain Phosphate and/or may have a pH between 6.5 and 8, which is favourable for the detection of the liver enzymes mentioned above.

In an embodiment:

- the blood-test disposable comprises an onboard control of activity of the catalysts, the onboard control comprising:

    - a dry control substrate containing oxaloacetate, and

    - a dry control pad containing the catalysts and the indicator, the control pad being distinct from the control substrate,

- the blood-test disposable is configured so that a liquid soaks the control substrate and the control pad when the blood-test disposable is inserted in the blood-test reader or when the blood sample is received in the blood-test disposable.

In the presence of pyruvate oxidase and peroxidase, oxaloacetate reacts to produce, inter alia, hydrogen peroxide. MAOS is then oxidized by the hydrogen peroxide and thus becomes colored, which is optically detected by the blood-test reader, thus confirming the catalysts (pyruvate oxidase and peroxidase) integrity and activity. So, this onboard control improves the reliability of the system (in particular, when a low liver enzyme concentration is measured, this control confirms that this result is not due to a catalysts deficiency).

One may note that pyruvate oxidase and peroxidase allows for the detection of either AST, or ALT (when combined with alpha-Ketoglutarate, and L-aspartic acid or L-alanine respectively). So, the integrity of a disposable configured to detect both AST (in a first reaction zone) and ALT (in a second detection zone) can be assessed, in a rather complete manner, with a single on-board control like the one presented above (instead of needing two distinct controls), which simplifies the structure of the disposable, and the quantity of liquid required for its operation.

The liquid mentioned above, that soaks the control substrate and the control pad when the blood-test disposable is inserted in the blood-test reader, may be a liquid buffer contained in a breakable blister. This blister may be configured to break and inject the buffer towards the control pad and substrate when the blood-test disposable is inserted into the blood-test reader. Employing such a buffer to achieve this integrity control, instead of employing the plasma sample drawn from the collected blood sample enables to reduce the volume of blood required for the liver enzyme measurement, which is highly beneficial.

In some embodiments, the system is further configured to allow for determining a platelets count in the blood sample or in another blood sample collected from the subject, and the control and processing system is programmed in order to determine the health condition, in step S3, taking also into account the platelets count.

In some embodiments of the system:

- the elastography device is configured to carry on also a measurement of an ultrasound attenuation parameter in liver, and

- the control and processing system is further programmed:

    - to acquire a value of the ultrasound attenuation parameter measured by the elastography device, in step S1, and
    - to determine the health condition of the liver of the subject taking also into account the value of the ultrasound attenuation parameter, in step S3.

In particular, the control and processing system may be programmed in order to compute, in step S3, a benchmark parameter, representative of a health condition of the liver of the subject, taking into account the value of the at least one mechanical parameter, the value of the ultrasound attenuation parameter and the value of the concentration of the at least one liver enzyme.

The at least one mechanical parameter, relative to the propagation of shear waves in liver, may be a liver stiffness, the ultrasound attenuation parameter may be an ultrasound attenuation coefficient per unit length, and the at least one liver enzyme may be AST. And the control and processing system may be programmed to compute the benchmark parameter as being equal or proportional to the exponential of X, divided by 1 plus the exponential of X, X being a linear combination of the logarithm of liver stiffness and the cube of the ultrasound attenuation coefficient, minus a correction term that is all the higher than the concentration of AST is small. This correction term may be proportional to 1 over the concentration of AST, for instance. In particular, the benchmark parameter could be the FAST parameter presented above, computed according to formula F1 (which corresponds to $X = -1.65 + 1.07 \times ln[LSM] + 2.66 \times 10^{-8} \times CAP^3 - 63.3/AST$).

Employing such a benchmark parameter, in particular when the AST concentration and the liver stiffness measurements are concomitant, allows for a very reliable discrimination between a healthy condition of the liver of the subject, and a poor/diseased one.

It will be appreciated that, according to the disclosed technology, the different embodiments presented above can be combined together, according to all technically possible combinations.

The disclosed technology also provides a method for determining a health condition of the liver of a subject, by means of a system comprising:

- an elastography device configured to measure at least one mechanical parameter relative to the propagation of shear waves in liver,
- a blood-test reader and a blood-test disposable associated to the blood test reader, the blood-test disposable being configured to receive a capillary blood sample and to be inserted in the blood test reader, the blood-test reader being configured to determine a concentration of at least one liver enzyme in the blood sample, wherein:

  - the blood-test disposable comprises:

    - a capillary tube for collecting the blood sample from the finger, and

    - reagents, appropriate to detect the at least one liver enzyme in the blood sample, and wherein

    - the blood-test reader is operatively connected to the elastography device, and

- a control and processing system, comprising at least a processor and a memory, configured to control the elastography device and the blood test reader,

the method comprising the following steps:

- S100 - a value of the mechanical parameter is measured using the elastography device, for the liver of the subject under examination,

- S1 - the control and processing system acquires the value of the at least one mechanical parameter, measured by the elastography device,

- S200 - a capillary blood sample is collected from the subject by means of the capillary tube, and the blood-test disposable is then introduced in the blood-test reader,

- S2 - the control and processing system acquires a value of a concentration of the at least one liver enzyme in a blood sample collected from the subject, measured by the blood test reader, and

- S3 - the control and processing system determines a health condition of the liver of the subject, taking into account both the value of the at least one mechanical parameter and the value of the concentration of the at least one liver enzyme, the health condition being represented by a health or disease stage identified among different stages of a given health condition classification, or being represented by a value of a benchmark parameter taking into account both the value of the at least one mechanical parameter and the value of the concentration of the at least one liver enzyme, or being represented both by the health or disease stage and by the value of the benchmark parameter, and outputs data representative of the health condition. In accordance with claim 14, the health condition is in form of a

EP 3 939 514 B1

value of a benchmark parameter, and it is the value of the benchmark parameter that is determined and output in step S3.

In one aspect of the present invention, steps S100 and S200 are executed within a single time frame having a maximum, preset duration. This maximum duration may be 30 minutes, or 15 or even 10 minutes.

In the method presented above, the control and processing system may output an error message when steps S100 and S200 are not executed within a single time frame having the maximum duration.

The features of the different embodiments of the system described above may apply also to the method for determining a health condition of the liver of a subject that has just been presented.

BRIEF DESCRIPTION OF THE DRAWINGS

Other characteristics and benefits of the disclosed technology will become clear from the description which is given below, by way of example and non-restrictively, in reference to the figures, in which:

Figure 1A schematically illustrate a first embodiment of a system for determining a health condition of the liver of a subject, in accordance with the disclosed technology;
Figure 1B represents the system of figure 1A in a more figurative way;
Figure 2 schematically illustrates a second embodiment of a system for determining a health condition of the liver of a subject, in accordance with the disclosed technology;
Figure 3 schematically illustrates a third embodiment of a system for determining a health condition of the liver of a subject, in accordance with the disclosed technology;
Figures 4 is a schematic perspective view of a blood-test disposable that may equip the system of any of figures 1A to 3;
Figures 5 and 6 are schematic bottom and top views of the blood-test disposable of figure 4;
Figure 7 is a schematic side view of detection pads of the blood-test disposable of figure 4;
Figure 8 is a schematic side view of an onboard control of the blood-test disposable of figure 4;
Figure 9 shows chemical reactions involved in a liver enzyme detection implemented in the blood-test disposable of figure 4;
Figure 10 is a flow-chart representing various steps of this liver enzyme detection;
Figure 11 and 12 represents steps of a first embodiment of a method for determining a health condition of the liver of a subject in accordance with the disclosed technology, that could be implemented by means of the system of any of figures 1A to 3;
Figure 13 represents schematically how different benchmark parameters can be selected and then computed to determine the health condition of the liver of the subject, in the method of figure 11;
Figure 14 shows in more detail a calculation and test sequence executed in an example of implementation of the method in Figure 11;
Figure 15 represents steps of a second embodiment of a method for determining a health condition of the liver of a subject in accordance with the disclosed technology, that could be implemented by means of the system of any of figures 1A to 3.

DETAILED DESCRIPTION

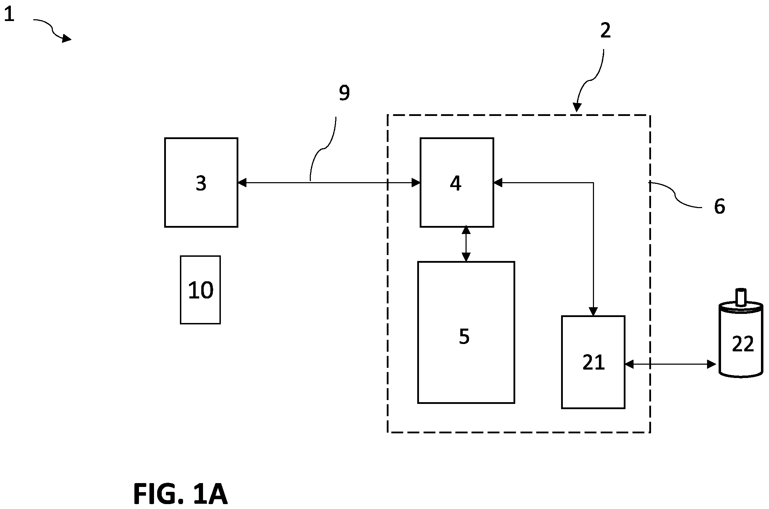

Figures 1A, 2 and 3 represent respectively a first, a second and a third embodiment of a system 1; 1'; 1" for determining a health condition of the liver of a subject.

In each of these embodiments, the system 1; 1'; 1" comprises:

- an elastography device 2; 2'; 2" configured to measure at least one mechanical parameter relative to the propagation of shear waves in liver, such as liver stiffness, and

- a blood-test reader 3, and a blood-test disposable 10 associated to the blood test reader 3, for determining a concentration of at least one liver enzyme in a blood sample collected from the subject under examination.

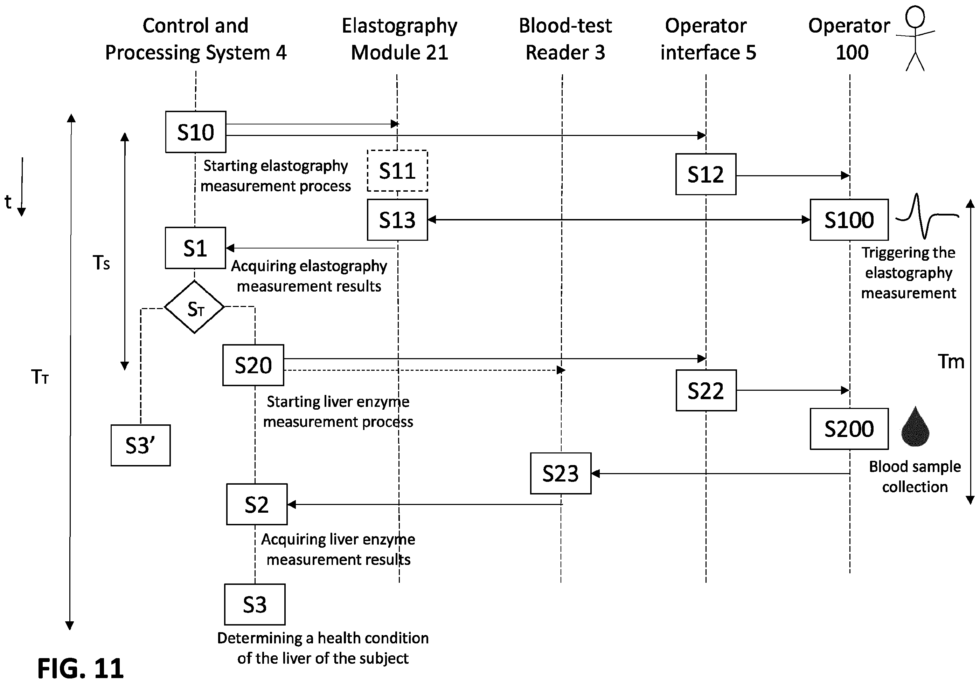

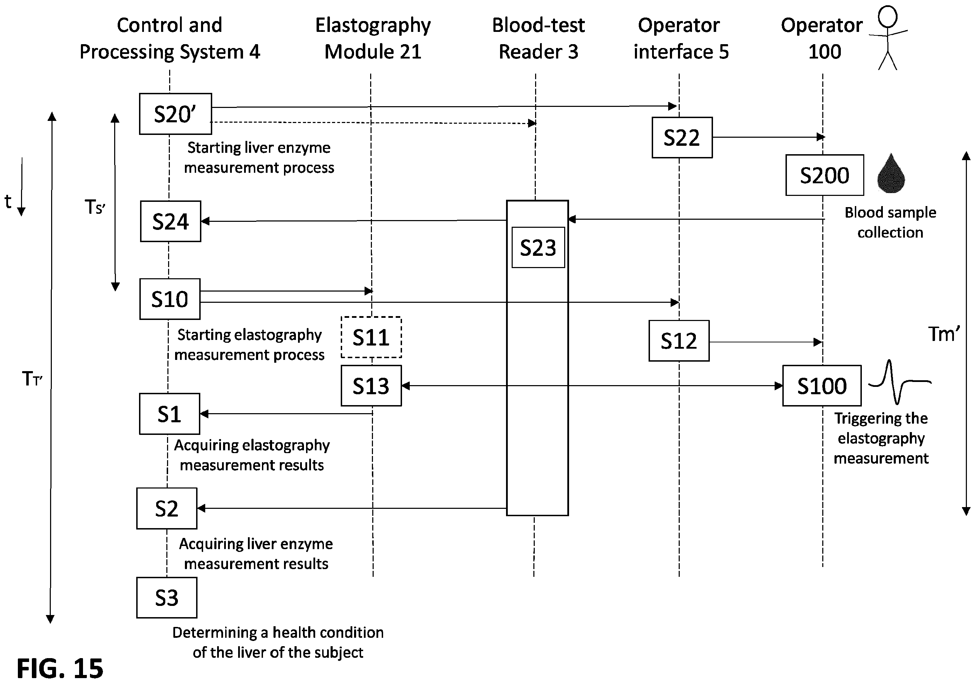

The system 1; 1'; 1" comprises also a control and processing system 4; 4'; 4", to control the elastography device 2; 2'; 2" and the blood-test reader 3 when examining the subject, for instance according to the examination process represented in figure 11 or 15.

The control and processing system 4; 4'; 4", which comprises at least a processor and a memory is programmed to determine a health condition of the liver of the subject, taking into account both:

- a value of the at least one mechanical parameter, measured by the elastography device 2; 2'; 2" during the examination of the subject, and

- a value of the concentration of the at least one liver enzyme, measured by the blood-test reader 3 during this examination.

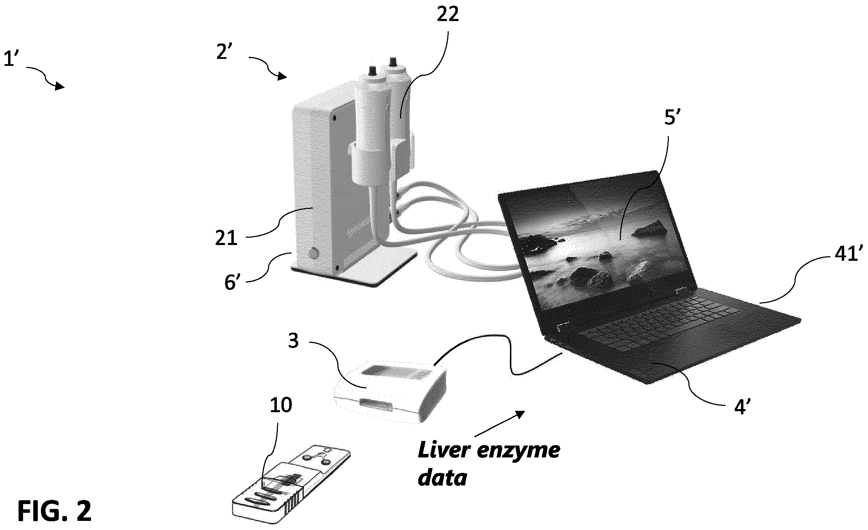

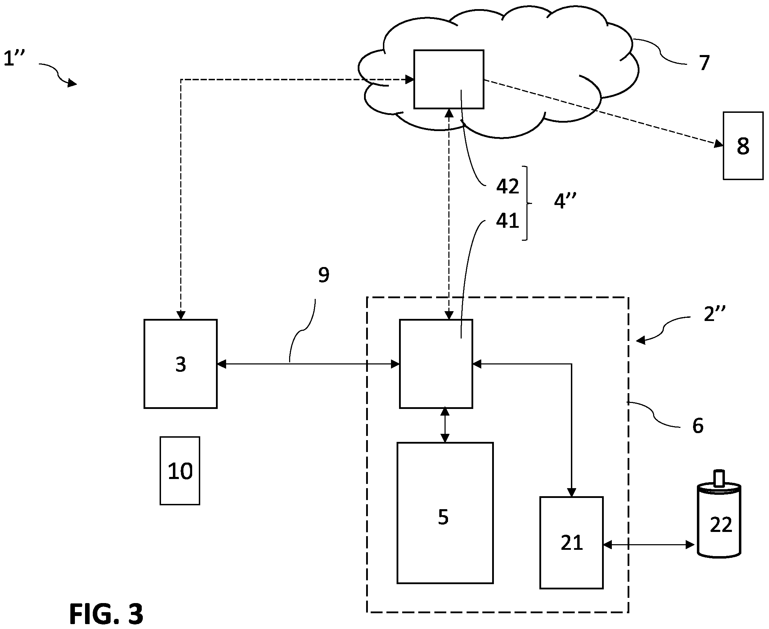

The main differences between the three embodiments of the system, 1; 1' and 1" (represented in figures 1A, 2 and 3 respectively) concerns the way the control and processing system 4; 4'; 4" is distributed within the system 1; 1'; 1". For instance, in the case of figure 1A, the control and processing system 4 is hosted in a same casing 6 as the elastography device 2 and is in fact realized in the form of a control unit of the elastography device 2. On the contrary, in the case of figure 2, the control and processing system 4' is a distinct electronic device, external both to a casing 6' of the elastography device 2' and to the one of the blood-test reader 3. And in figure 3, part of the control and processing system 4" is implemented by means of delocalized, distant computing resources 7 (such as a "cloud").

Anyhow, these three embodiments of the system 1; 1'; 1" have many features in common. So, identical or corresponding elements of these different embodiments may be described just once and may be identified with the same reference symbols/numbers.

In each of these embodiments, the blood-test reader 3 is operatively connected to the elastography device 2; 2'; 2". This connection can be achieved directly, with no intermediate system. It could also be achieved via an intermediate device, like in the case of figure 2, in which the elastography device 2' is operatively connected to the blood-test reader 3 via the control and processing system 4'.

Anyhow, the connection 9 between the elastography device 2; 2'; 2" and the blood-test reader 3 allows for a coordinated, for instance concomitant operation of these two devices.

The connection 9 may be employed for instance to transmit commands or execution requests from the elastography device 2 to the blood-test reader 3. In this case, the elastography device 2 and blood-test reader 3 may be programmed to operate according to a master-slave model, the elastography device 2 being the master and giving orders to the blood-test reader 3, which executes them and, in response, sends acknowledgement data, measurements results, data relative to a liver enzyme measurement process and/or data representative of a status of the blood-test reader 3.

The connection between the elastography device 2' and the blood test-reader 3 may also allow for the transmission, to the blood-test reader, of commands or execution requests that are not determined directly by the elastography device 2', but that are however determined on the basis of data transmitted by the elastography device 2' (such as liver stiffness measurements or status data). In particular, these commands or requests may be determined by the control and processing system 4' on the basis of data received from the elastography device 2', the control and processing system 4' thus playing an active role in the connection of the elastography device 2' with the blood-test reader 3 (this connection being then a somehow composite, and active connection). Conversely, the elastography device 2' could receive commands or execution requests determined (by the control and processing system 4') on the basis of data received from the blood-test reader 3.

The connection 9 between the elastography device 2; 2'; 2" and the blood-test reader 3 may be implemented by means of a wire or wireless link, according to an USB, a Firewire, a Bluetooth, a 6LoWPAN, a ZigBee, a Z-Wave, a Sigfox or another protocol.

As mentioned above, the connection 9 between the elastography device 2; 2'; 2" and the blood-test reader 3 allows for a coordinated, for instance concomitant operation of these two devices. And the control and processing system 4; 4'; 4" may, like here, be programmed to control the elastography device and the blood-test reader so that they execute respectively an elastography measurement process and a liver enzyme measurement process in a coordinated, more particularly in a concomitant manner (which is made possible by the connection mentioned above).

As mentioned in the summary section above, achieving concomitant measurements of a mechanical parameter such as liver stiffness, and of at least one liver enzyme concentration makes the determination of the health condition of the liver of the subject much more reliable and reproducible.

The particular blood-test disposable 10 and reader 3 employed to measure the concentration of at least one liver enzyme in the subject's blood participates in this improvement. They indeed allow for determining the concentration of at least one liver enzyme in the subject's blood accurately, quickly, in situ, and from a very limited amount of capillary blood collected from the subject. These features, in particular the fact that the volume of blood that has to be collected is minute, simplifies the overall procedure and helps greatly to achieve concomitant measurements of the liver stiffness and of a liver enzyme level.

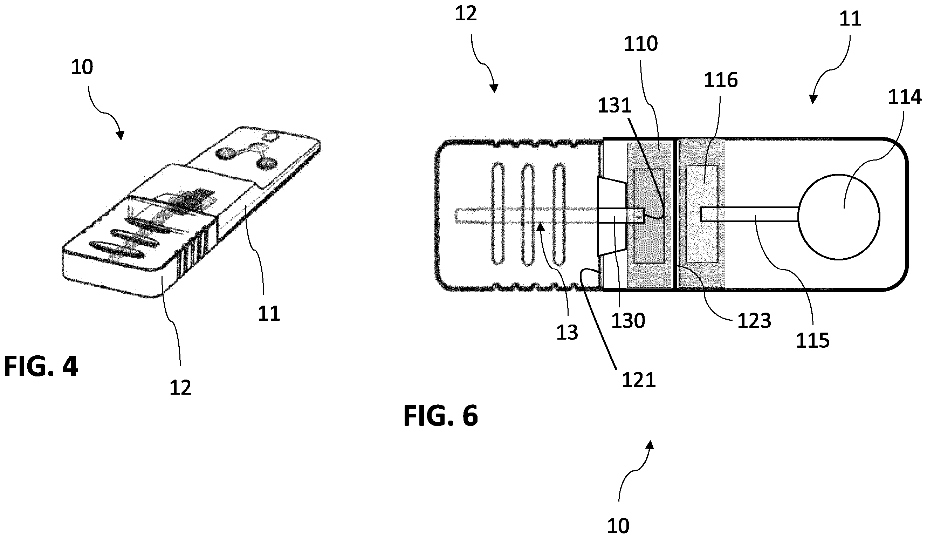

The general structure of the system 1; 1'; 1" is now presented in more detail, in reference to figures 1A to 3. The particular blood-test disposable 10 and reader 3 employed in this system 1; 1'; 1" will be presented further, in reference to figures 4 to 10. And then, two different embodiments of a method for determining the health condition of the liver of the subject will be presented, in reference to figures 11 and 15 respectively. Each of these two methods can be implemented by means of any of the three embodiments of the system 1; 1' and 1" mentioned above. And the control and processing system 4; 4'; 4" of any of these systems 1; 1'; 1" may be programmed to execute one or the other of these methods.

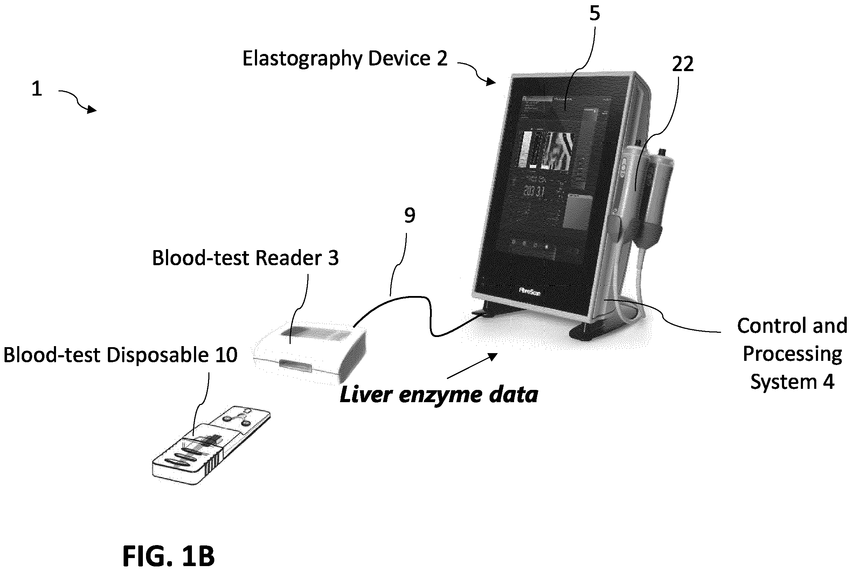

In the system 1 of figures 1A and 1B, the control and processing system 4 is part of the elastography device 2 and plays the role of a control unit of the elastography device 2 (and, in addition, it controls the blood-test reader 3).

As mentioned above, the elastography device 2 is configured to measure at least one mechanical parameter relative to the propagation of shear waves in liver. Here, this mechanical parameter is a quantity related to the liver stiffness, such as the propagation speed of shear waves Vs, the shear modulus of liver tissue or its Young's modulus E. This parameter is designated here as the liver stiffness or LSM (for "Liver Stiffness Measurement"). However, in other embodiments, the mechanical parameter mentioned above could be a quantity related to low frequency (e.g. lower than 500 Hz) shear wave attenuation in the tissue, like viscosity.

The elastography device 2 may be configured to measure the at least one mechanical parameter by transient elastography, for example.

Here, the elastography device 2 is configured also to measure an ultrasound attenuation parameter in liver, more precisely an ultrasound attenuation coefficient per unit length, referred to as a controlled attenuation parameter or "CAP". This ultrasound attenuation coefficient is representative of the attenuation of high frequency ultrasound waves in the organ under examination (these ultrasound waves having typically a central frequency of a few MHz - for instance 2 to 5 MHz).

The elastography device 2 comprises an elastography module 21 configured to drive a probe 22 and for receiving signals acquired by the probe 22.

The probe 22 comprises at least a vibrator for generating a shear wave, and an ultrasound transducer for transmitting ultrasound shots and receiving corresponding echo signal to track how the liver of the subject is moved by the shear wave generated by the vibrator. The probe 22 is held against the subject's skin, during the liver stiffness measurement.

The elastography module 21 comprises an ultrasound front end comprising electronic modules for generating the ultrasound signals to be transmitted, and for acquiring and preprocessing the ultrasound echo signals received by the ultrasound transducer of the probe 22. The elastography module 21 comprises also a motion actuator servo controller for driving the vibrator of the probe 22.

The elastography module 21 may also comprise special purpose logic circuitry (e.g. an FPGA, an ASIC - application specific integrated circuit -, or an other kind of programmable microcircuit), for processing the acquired echo signals in order to derive a value of the mechanical parameter to be measured (e.g.: liver stiffness). This special purpose logic circuitry could also be included in the control and processing system 4, instead of being included in the elastography module 21.

The control and processing system 4 is connected to the elastography module 21 and to an operator interface 5, for instance a display screen. During the measurement of the mechanical parameter mentioned above, the elastography module 21 and the operator interface 5 are controlled by the control and processing system 4. For instance, the control and processing system 4 controls the operator interface 5 so that it displays guiding information to help the operator positioning the probe in front of the subject's liver, and displays measurement results (such as an elastogram and a stiffness value finally obtained) once acquired.

As already mentioned, the control and processing system 4 comprises at least a processor and a memory coupled to the processor. More generally, it comprises an electric circuitry for processing data and for transmitting and receiving data. The memory comprises a physical non-transitory (non-volatile) memory module for storing machine executable instructions to be executed by the processor in order to carry out the functions of the control and processing system 4. It may also comprise a RAM memory for storing signal data and instructions during the system operation.

In the system 1 of figure 1A, the control and processing system 4 and the elastography module 21 are both hosted in the casing 6 of the elastography device 2. The operator interface 5 could be integrated to this casing 6 or could be realized in the form of a distinct, remote device. The elastography device 2 and the blood-test reader 3 may be located in a same room, or may be separated from each other by less than 15 meters, to avoid transferring the subject or the blood sample from one place to another, in order to avoid transfer time or errors.

As already mentioned, the control and processing system 4 is operatively connected also to the blood-test reader 3, through the connection 9 mentioned above. And the control and processing system 4 is programmed not only to supervise the execution, by the elastography device 2, of the measurement process of the mechanical parameter mentioned above. It is also programmed to control the blood-test reader 3 in order to trigger a liver enzyme measurement process in a coordinated, for instance concomitant manner with the mechanical parameter measurement. For instance, the control and processing system 4 may be programmed to launch the liver enzyme measurement process depending on a liver stiffness value obtained first (the liver enzyme measurement process being launched immediately or almost immediately after this stiffness value is obtained). It may also be programmed to launch the liver enzyme measurement process and the mechanical parameter measurement process almost in parallel, with only a short delay between their respective start (for instance less than 5 minutes, or less than 15 minutes).

The control and processing system 4 may be programmed also to check, a posteriori, once these measurement processes are completed, that time gap between a measurement moment of the mechanical parameter mentioned above, and a measurement moment of the concentration of one or more liver enzyme in the subject's sample, is below a given duration threshold. If this time gap is above this duration threshold, the control and processing system 4 may output an error message specifying that the health condition assessment of the liver of the subject failed, or that the health condition assessment of the liver of the subject may not be reliable and/or it may skip the determination or the transmission of this health condition.

Anyhow, as already mentioned, the control and processing system 4 is programmed to determine a health condition of the liver of the subject, taking into account both a value of the mechanical parameter mentioned above, measured by the elastography device 2, and a value of the concentration of one or more liver enzymes, measured by the blood-test reader 3 (even if this determination may be carried on, or not, depending some conditions, based for instance on the results of a preliminary stiffness measurement).

Different ways to determine the health condition of the liver of the subject, from these two measurements, and possibly from other measurements too, will be presented when describing the methods of figures 11 and 15.

The control and processing system 4 of the system 1 of figure 1A may be programmed more specifically to control the blood-test reader 3 and the elastography device 2 according to the method of figure 11, or according to the method of figure 15. In particular, the control and processing system 4 may be programmed to execute the steps S10, S1, S20, S2 and then S3 (or, alternatively, S3') of the method of figure 11. Or to execute the steps S20, S10, S1, S2 and then S3 of the method of figure 15.

The system 1' of figure 2 is similar to the one of figure 1A, but in this second embodiment, the control and processing system 4' is distinct from the elastography device 2'.

The elastography device 2' comprises the elastography module 21 and the probe 22 presented above. The elastography module 21 is hosted in a casing 6' distinct from the casing 41' of the control and processing system 4' to which it connected to. An operator interface 5', such as the one, 5, presented above is connected to the control and processing system 4'.

In this second embodiment, the control and processing system 4' may be an electronic logic circuit or system (with one or more processor and one or memories) of a personal computer such as a laptop or tablet computer, or of a smartphone operatively connected to both the elastography device 2' and the blood-test reader 3.

The blood-test reader 3 and disposable 10 are identical to the ones of the system 1 of figure 1A, and the blood-test reader 3 is connected to the control and processing system 4' just as in the system 1 of figure 1A.

An additional, optional connection (not represented in figure 2) could also link the elastography device 2' (more specifically, its elastography module 21) directly to the blood-test reader 3 (without passing through the control and processing system 4').

The system 1" of figure 3 is similar to the one of figure 1A, but in this third embodiment, part of the control and processing system 4" is implemented by means of delocalized, distant computing resources 7 (such as a "cloud").

In this third embodiment, the control and processing system 4" comprises:

- a first part 41, configured to control the elastography device 2" and the blood-test reader 3 and to supervise the measurement processes, and

- a second part 42, configured to determine the health condition of the liver of the subject, in particular by computing a benchmark parameter (a score).

The first part 41 is implemented locally. More particularly, it is integrated to the elastography device 2" and hosted in the casing 6 of the elastography device 2". On the contrary, the second part 42 of the control and processing system, for instance a delocalized computing service and data base, is remote and implemented by means of distant, and possibly distributed computing resources 7 (distant meaning that at least a part of these resources is separated from the elastography device by 1 kilometer at least).

The first and second parts 41 and 42 of the control and processing system 4" are operatively connected to each other, and so they can exchange data and instructions. The blood-test reader 3 may also be connected directly to the second, remote part 42 of the control and processing system 4", without passing through the first part 41 (hosted in the elastography device), which allows for transferring liver enzyme measurements directly to the second part 42 of the control and processing system 4", devoted more specifically to data processing and computations.

The second part 42 of the control and processing system 4" can be programmed in order to transmit data representative of the health condition of the subject, after having determined this data:

- to the first part 41 of the control and processing system 4", so that this health condition can be transmitted to the operator by means of the operator interface 5, and/or

- to a remote device 8 such as a personal computer of smartphone, and/or

- to a remote data base, for storage.

Apart from the differences mentioned above, the third embodiment of the system 1" is identical, or at least similar to the first one. In particular, the elastography device 2" includes, in the same casing 6, the elastography module 21 (which is connected to the probe 22) and the first part 41 (control part) of the control and processing system 4". This first part 41 of the control and processing system 4" is connected, by connection 9, to the blood-test reader 3 (which may be the same as in figures 1A and 2).

The blood-test disposable 10 of the system 1 of figure 1A is presented now in more detail, in reference to figures 4 to 10. As mentioned above, this blood-test disposable 10 could be employed indifferently in any of the three embodiments of the system 1; 1'; 1" presented above.

The blood-test disposable 10 comprises a capillary tube 13 for collecting a blood sample from the subject. To this end, the skin of a finger, or possibly of 2 or 3 fingers of the subject is punctured, for instance by means of a lancet. The droplet, or droplets of capillary blood thus obtained are then collected, by means of the capillary tube. Here, the capillary tube 13 is fixed, more precisely irremovably fixed to a part of the blood test disposable (it is not a removable, replaceable piece).

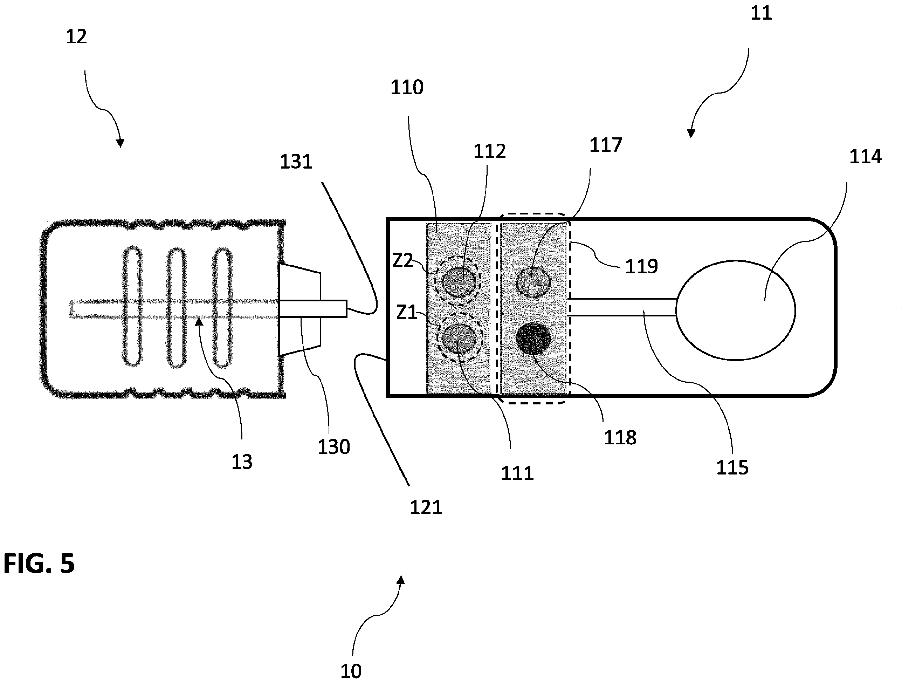

As shown in figure 5, the blood test disposable 10 comprises two distinct parts, namely:

- a cartridge 11, containing reagents appropriate to detect at least one liver enzyme, and

- a detachable plug 12 (in figure 5, the plug 12 is represented detached from the cartridge 11).

The capillary tube 13 is fixed to the detachable plug 12. But in other, non-represented embodiments, the capillary tube could be fixed to the cartridge instead of being fixed to the plug.

The plug 12 and the cartridge 11 are configured so that the plug 12 can be detached from the cartridge 11 and then re-plugged onto the cartridge 11.

To this end, the plug 12 and cartridge 11 may be provided respectively with male and female fastening elements (or conversely, with female and male elements), such as a rib (on the plug) and a groove (on the cartridge) having complementary shapes, or such as elastic teeth or clips and corresponding recesses or holes.

Here, the plug 12 is a kind of cap, removably fixed to an end face 121 of the cartridge, namely, its back face (the front face of the cartridge being the one that is introduced first in the reader 3, when the disposable 10 is introduced in the reader 3).

The capillary tube 13 protrudes from the plug 12, to allow for collecting blood from the subject. It extends also inside the plug 12, to provide a receiving volume large enough for receiving a blood sample between 20 and 50 microliters, for instance of 25 +/- 10 microliters.

When the plug 12 is plugged onto the cartridge 11 (like in figure 6), the outer part 130 of the capillary tube 13, that is the part of the capillary tube 13 that protrudes from the plug 12, enters into the cartridge 11. The capillary tube 13 then extends entirely inside the blood-test disposable 10 (in other words, the capillary tube 13 is then hosted entirely inside the blood-test disposable 10).

To collect the blood sample, the operator (who could be the subject under examination himself) punctures the skin of at least one finger of the subject, for instance with a lancet. The operator also detaches the plug 12 from the cartridge 11 and then collects the droplet (or droplets) of capillary blood thus obtained, with an end 131 of the capillary tube 13. Then, once the capillary tube 13 (and, possibly, an auxiliary reservoir) has been filed by capillary blood, the operator re-plugs the plug 12 onto the cartridge 11. To collect the appropriate volume of blood, that is, here, to fill the capillary tube 13, the operator may puncture more than one finger of the subject, for instance 2 or 3.

The blood-test disposable 10 is configured so that the blood sample, thus collected by means of the capillary tube 13, comes into contact with the reagents contained in the cartridge 11.

In the embodiment represented in the figures, the blood sample collected by means of the capillary tube 13 gets out of this tube (to flow into the cartridge and to mix with the reagents) by the same end 131 of the tube as the one employed to collect the blood sample. And here, the plug 12 is configured so that plugging it onto the cartridge 11 causes a pressure increase that pushes the blood sample, contained in the capillary tube, towards the cartridge 11, inside the cartridge 11. To this end, the plug 12 may have a deformable body that is squeezed when an operator pushes the plug 12 to plug onto the cartridge, for instance.

Once out of the capillary tube 13, the blood sample may be brought into contact with the reagents mentioned above, and more generally routed in the different parts of the cartridge employed for enzyme detection and for control, by means of fluidic circuitry (such as microfluidic circuitry) comprising one or more pipes and/or junctions. It may also reach these reagents directly, or by soaking and traversing one or more membranes or substrates (without going through pipes or other circuitry).

As represented in figure 6, when the plug 12 is plugged onto the cartridge 11, the output end 131 of the capillary tube 13 is located just above a filtering membrane 110. This filtering membrane 110 is appropriate to filter blood red cells out of the blood sample, in order to obtain a plasma sample.

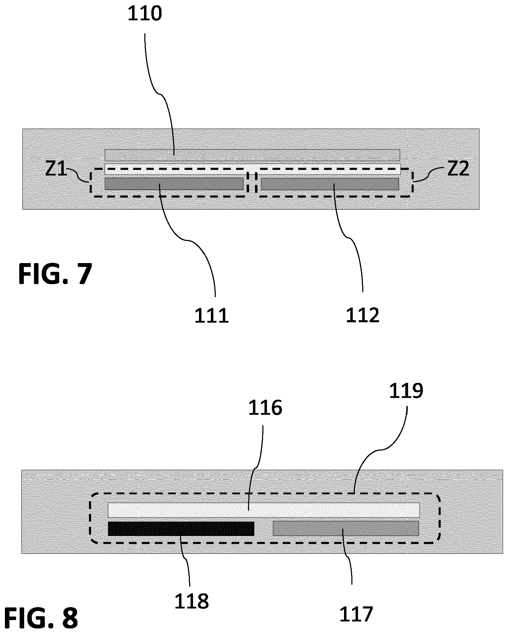

The cartridge 11 comprises also, in a first reaction zone Z1 located below the filtering membrane 110 (on the other side of the membrane than the capillary tube output 131), a first detection pad 111 that comprises the reagents mentioned above (appropriate to detect optically one or more liver enzyme), in a dry form (figures 5 and 7). Here, the first detection pad 111 comprises more particularly reagents appropriate to detect AST. The cartridge 11 comprises also, in a second reaction zone Z2 located below the filtering membrane 110, a second detection pad 112 that comprises reagents appropriate to detect another liver enzyme, namely ALT. The first and second detection pads 111 and 112 are located side by side, below the filtering membrane 110, for instance in the form of two distinct reagents dots (figure 5).

So, after passing through the filtering membrane 110, a part of the blood sample, which has in fact become more of a plasma sample due to filtration, reaches and soaks the first detection pad 111. And another part of the filtered blood sample reaches and soaks the second detection pad 112.

When in contact with AST, the reagents contained in the first detection pad 111 produce, as a result of one or more chemical reactions, a product (here hydrogen peroxide) which reacts with an indicator (here, MAOS Trinder reagent) that becomes colored in the presence of this product. So, thanks to this reaction, or reactions, in the presence of AST, this indicator becomes colored, which allows for the liver enzyme detection.

The color change of the indicator is detected optically by the blood-test reader 3, which is configured to measure a light reflectance, more particularly a diffuse light reflectance at the first reaction zone Z1. To this end, the blood-test reader 3 is equipped with a light source and a light detector. Here, the light source, for instance a Light-Emitting Diode, has an emission spectrum appropriate to detect the color change of the indicator, even without spectral filtering of the light reflected by the first reaction zone Z1 of the blood-test disposable 10. For instance, if the indicator in its colored form absorb selectively yellow light, the light source may emit mainly yellow light (in other words, the emission spectrum of the light source may coincide at least partially with the absorption spectrum of the indicator in its colored form).

One may note that the expression "light reflectance" designates any quantity representative of the intensity or spectral intensity or power of the light reflected by an object or a surface, as compared to the power or intensity or spectral intensity with which this object or surface is lighted. In particular, the expression "light reflectance" may designate a reflectivity, a reflectance coefficient, a spectral reflectance, or an incident-to-reflected luminous intensity ratio for a light whose spectrum is mainly contained in a given, limited wavelength range. And the light reflectance of zone Z1 may be measured or estimated by comparing a luminous intensity of light reflected by this zone with a luminous intensity of light reflected by another, reference zone (employed as a "blank").

The blood-test reader 3 is calibrated and programmed to determine an AST concentration in the blood sample collected from the subject, from the light reflectance measurement mentioned above.

Similarly, when in contact with ALT, the reagents contained in the second detection pad 112 produce, as a result of one or more chemical reactions, a product (again hydrogen peroxide) which reacts with an indicator (again, MAOS Trinder reagent) that becomes colored in the presence of this product.

The blood-test reader 3 is configured to measure also a light reflectance at the second reaction zone Z2 (for instance in the same way as for measuring the light reflectance at the first reaction zone Z1), and for determining an ALT concentration in the blood sample from this measurement.

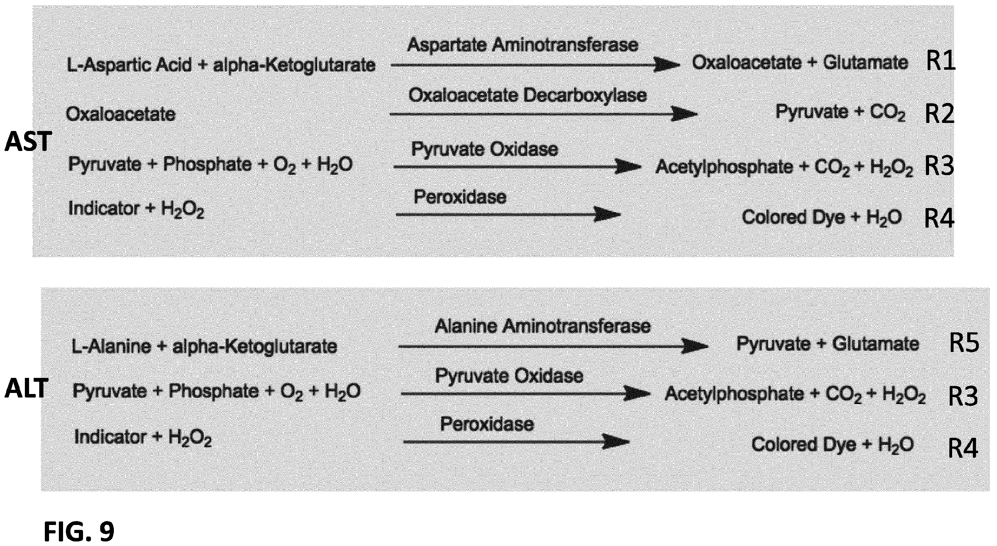

The kind of reagents and the chemical reactions involved in this enzyme detection are now presented in more detail, in reference to figure 9.

The first detection pad 111 (for AST detection) comprises L-Aspartic acid and alpha-Ketoglutarate. When part of the filtered blood sample, that contains AST, comes into contact with this pad, the L-Aspartic acid and the alpha-Ketoglutarate react together, AST playing the role of a catalyst (figure 9). This first reaction, R1, produces oxaloacetate, which then decomposes into Pyruvate and carbon dioxide, in reaction R2.

The first detection pad 111 contains also catalysts, namely Pyruvate Oxidase and Peroxidase. The Pyruvate produced by reaction R2 reacts with Phosphate, Oxygen and water contained in the filtered blood sample (sample that is possibly mixed with a buffer) to produce acetyl phosphate, carbon dioxide and hydrogen peroxide, during a reaction referred to as R3. Reaction R3 is catalyzed by Pyruvate Oxidase.

Here, the indicator contained in the first detection pad 111 becomes colored when oxidized. So, in presence of the hydrogen peroxide produced by reaction R3, the indicator oxidizes and becomes colored, during reaction R4. Reaction R4 is catalyzed by peroxidase. The indicator may for instance be MAOS Trinder reagent (whose detailed formula has been given above). Indeed, this indicator turns out to allow for a sensitive and reliable enzyme concentration measurement. In this case, the emission spectrum of the light source employed to detect MAOS state change may contain mainly orange light (his spectrum spanning mainly around a wavelength of 610 nanometers), or may be filtered, so that the reflectance measurement is carried on in a wavelength range spanning mainly around a wavelength of 610 nanometers, for instance comprised mainly between 550 and 650 nanometers.

Here, the first detection pad 111 comprises two distinct sub-pads, namely an upper pad containing the reagents (L-Aspartic acid and alpha-Ketoglutarate), and a lower pad containing the catalysts (Pyruvate Oxidase and Peroxidase) and the indicator (MAOS). The lower pad is located under the upper pad. The filtered blood sample (that is, the plasma sample) first reaches the upper, reagents pad, and then, once pyruvate has been produced, flows or otherwise migrate to reach the lower, catalysts and indicator pad. The upper pad may be realized in the form of a substrate, extending over the lower pad (covering an area wider than the one of the lower pad). However, in some embodiments, the reagents, the catalysts and the indicator could be mixed in a single dry detection pad instead of being separated from each other (for a better preservation), or they could be distributed in distinct pads in a different manner than the one presented above.

The second detection pad 112 (for ALT detection) comprises L-Alanine (instead of L-Aspartic acid) and alpha-Ketoglutarate. It comprises also the same catalysts and indicator as in the first detection pad 111, namely pyruvate oxidase and peroxidase, and MAOS Trinder reagent. Here, the second detection pad 112 comprises an upper pad containing the reagents (L-Alanine and alpha-Ketoglutarate), and a lower pad containing the catalysts (Pyruvate Oxidase and Peroxidase) and the indicator (MAOS), similarly to the first detection pad 111.

The reactions occurring in the second detection pad 112, when a part of the filtered blood sample soaks it, are the following. First, the L-Alanine and the alpha-Ketoglutarate react together, ALT playing the role of a catalyst (reaction R5). This reaction, R5, produces Pyruvate and Glutamate. Pyruvate reacts then with Phosphate, Oxygen and water contained in the filtered blood sample (which is possibly mixed with a buffer) to produce acetyl phosphate, carbon dioxide and hydrogen peroxide, during a reaction that is the same as reaction R3 described above (involved in the detection of AST). The hydrogen peroxide produced by reaction R3 then oxidizes the indicator, which becomes colored, during the same reaction as reaction R4, presented above.

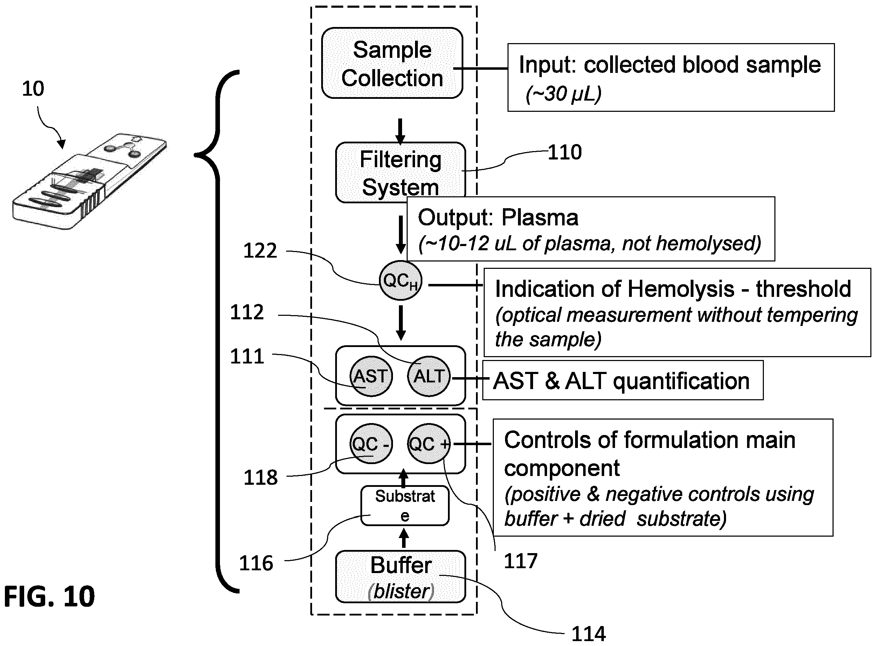

Optionally, the blood-test disposable 10 may also comprise a hemolysis check optical port, located along the path followed by the filtered blood sample, between the filtering membrane and the first reaction zone, as represented schematically in figure 10 (in figure 10, this optional element is identified by the reference number 122). This optical port may be realized in the form of a window with a passage for the filtered blood sample behind. To check whether the blood sample is noticeably hemolyzed or not, the blood-test reader would then be configured to measure light reflectance and/or transmittance, here light reflectance at the hemolysis check optical port, in a wavelength range appropriate to detect hemoglobin. For instance, this light reflectance may be measured in a wavelength range spanning mainly around 410 nanometers, for instance comprises mainly between 390 and 430 nanometers. This light reflectance measurement may be achieved by means of a Light Emitting Diode emitting blue light and by means of a light detector such as a photodiode.

The blood-test reader 3 may be programmed to test, from said reflectance and/or transmittance measurement, whether the filtered blood sample noticeably absorbs light in the wavelength range mentioned above (which indicates that it is noticeably hemolyzed), for instance by testing whether the light reflectance at the hemolysis check port passes a given threshold. And when it passes the threshold, the blood-test reader may emit an error message specifying that the liver enzyme concentration measurement failed. This error message may be transmitted by the blood-test reader so that the operator can directly be aware of this failure, for instance by emitting audible beeps, by lighting a specific light indicator, of by displaying an error message on a display screen of the blood-test reader. The blood-test reader may also be configured to transmit this error message to the control and processing system 4. Anyhow, when the blood-test reader 3 has determined that the filtered blood sample noticeably absorbs light in the wavelength range mentioned above, it inhibits the transmission of the liver enzymes concentrations measurements (it does not transmit or otherwise outputs the results of these measurements). Indeed, a high absorption in the wavelength range mentioned above indicates that the blood sample is noticeably hemolyzed, which modifies substantially its color and is thus likely to cause liver enzyme measurement errors (as the liver enzymes are detected by means of a colored indicator). The reliability of the liver enzymes measurements is thus improved, thanks to the hemolysis check port of the blood-test reader 10, and associated detection system of the blood-test reader 3.

The blood-test disposable 10 comprises also an onboard control of activity of the catalysts mentioned above, 119 (figures 5 and 8). This activity control is based on the same colorimetric detection scheme as for the liver enzymes detection, that is the reactions R3 and R4 presented above. To check the activity and integrity of the catalysts and indicator involved in these detection reactions, the onboard control 119 comprises oxaloacetate, which is expected to be detected by these catalysts and indicator (see reactions R2 to R4).

The onboard control 119 comprises more particularly:

- a dry control substrate 116 containing oxaloacetate, and

- a dry control pad 117 containing the catalysts mentioned above (that is Pyruvate Oxidase and Peroxidase), and the indicator (here, MAOS).

The control substrate 116 and the control pad 117 are distinct from each other, so that the oxaloacetate and the catalysts/indicator do no mix together (and so do not react with each other) during storage of the disposable 10. Here, the control pad 117 is located below the control substrate 116.

The cartridge 11 further comprises a breakable blister 114 configured to break when the blood-test disposable 10 is inserted into the blood-test reader 3. The blister 114 is in fluidic connection with the on-board control 119, here by means of a supply tube 115. The supply tube 115 extends from the blister 114 to an output end of the tube that is located just above the control substrate 116 (on another side of the control substrate than the control pad 117). When the blister 114 breaks, due to the insertion of the disposable 10 into the reader 3, the buffer flows into the supply tube 115 to reach the on-board controls, where it soaks the control substrate 116 and then the control pad 117, thus mixing the oxaloacetate of the control substrate 116 with the catalysts and indicator of the control pad 117. Reactions R2 to R4 then occur and the color of the indictor changes, except if the catalysts or indicator are defective. This change of color is detected optically by the blood-test reader 3.

For a more reliable and sensitive detection of this change of color, the on-board control 119 comprises a reference pad 118, whose color does not change in the presence of oxaloacetate, and that is used to record a "blank", reference value of light reflectance. Light reflection characteristics of the reference pad 118 are close, for instance identical within 20%, or even within 10% (at least in the visible range or in part of the visible range), to the ones of the control pad 117 when the color of the indicator hasn't changed yet. The reference pad 118 may for instance be identical to the control pad 117 except that it is devoid of the catalysts mentioned above (pyruvate oxidase and peroxidase) and/or of the indicator.

The liquid buffer (initially contained in the blister 114) is an aqueous solution containing Phosphate. It may have a pH comprised between 6 and 8, or even between 6.5 and 7.5. It may comprise TRIS, the concentration of TRIS being between 0.05 and 1 mole/liter, or even between 0.1 and 0.5 mole/liter.

The cartridge 11 may be configured so that the buffer not only flows through the control substrate 116, once the blister has been broken, but also reaches the detection area where the plasma sample mixes with the reagents and catalysts, the buffer then mixing with the plasma sample. This is beneficial, as the kind of buffer presented above (TRIS buffer, having the concentration and pH mentioned above) contributes to a fast and accurate detection of AST and ALT, when mixed with a plasma sample. In particular, the cartridge may comprise a permeable membrane 123 separating:

- a front part of the cartridge, containing the blister 114 and the onboard control 119, from

- a rear part of the cartridge, containing the detection pads 111 and 112, and in which the capillary tube 13 penetrates when the plug 12 is plugged onto the cartridge 11.

Figure 10 summarizes, as a flowchart, the different steps involved in the detection of liver enzymes and in the control of activity of the catalysts and indicator.

The blood-test disposable 10 and associated reader 3 presented above are configured specifically to detect AST and ALT.

Still, in other embodiments (not represented in the figures), the blood-test disposable and reader could be configured to measure the chemical activity of just one of these two enzymes (instead of both), for instance just AST. Or they could be configured to measure the chemical activity of another liver enzyme, like GGT, or to measure the chemical activity of AST and GGT, or of AST, ALT and GGT.

In some embodiments, the blood-test disposable and reader could be configured to measure also platelets count in the collected blood sample, in addition to AST (and ALT) chemical activity. To measure platelets count, a part of the blood sample collected by the capillary tube would be kept unfiltered, and a longer cartridge could be used to leave some place, between the capillary tube output and the filtering membrane, for platelets measurement elements.

In some embodiments, the system could also comprise another Point of Care device, configured to determine platelets concentration in a blood sample collected from the subject, in addition to the blood-test reader 3 and associated disposable 10 (that are configured to determine AST/ALT concentration in the subject's blood).

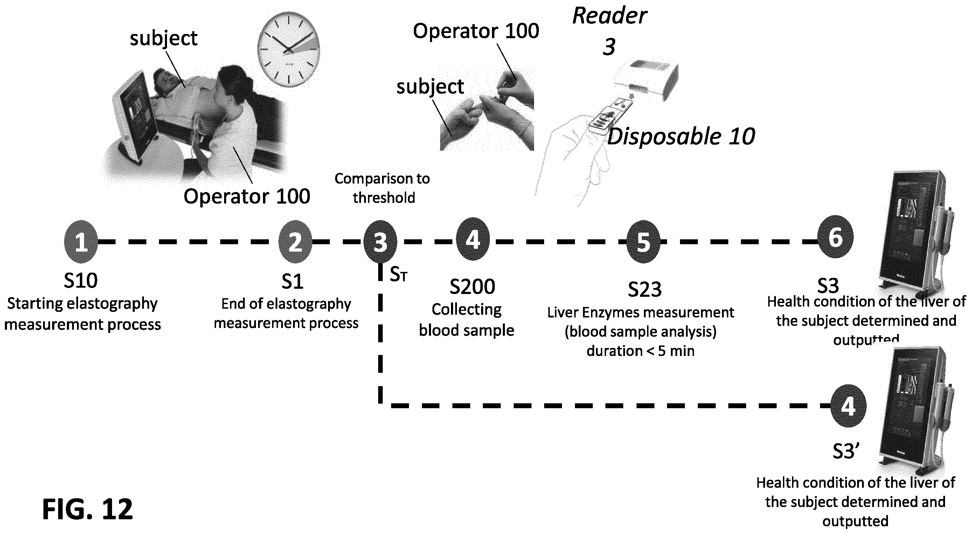

Figure 11 represents a first embodiment of a method for determining a health condition of the liver of a subject, as a flowchart. Figure 12 represents the same method as in figure 11, in a simplified but somehow more figurative and illustrative way.

In this method, the control and processing system 4 controls the elastography device 2 and the blood-test reader 3 so that the mechanical parameter is measured first (in steps S10 to S1). Then, depending on the value of the mechanical parameter thus measured, a blood sample is collected from the subject and liver enzymes concentrations are measured (in steps S20 to S2), or not. In this first embodiment, the blood test is carried on only when it is expected to improve the determination of the health condition of the liver of the subject.

In figure 11, different steps of this method are represented over time t. The direction in which the time t is elapsed corresponds to the vertical downward direction, in this figure. The steps in question are each vertically aligned with the entity executing the step considered. For instance, step S1 is executed by the control and processing system 4 while step S100 is executed by the operator 100.

The method starts with step S10. In step S10, the control and processing system 4 controls the elastography device 2, in particular the elastography module 21, so that it starts an elastography measurement process.

In response, in step S11, the elastography module 21 generates signals (in particular ultrasound signals) appropriate to probe the part of the subject's body against which the probe 22 is placed, in order to guide the operator 100 and to help him placing the probe 22 in front of the liver of the subject. The operator interface 5 transmits to the operator 100 (here by displaying them) guiding information thus obtained, such as A-mode ultrasound images, and other information useful for the elastography measurement (such as a contact force exerted on a tip of the probe 22), in step S12. In step S12, the operator interface 5 may prompt the operator to carry on an elastography measurement.

Then, in step S100, the operator 100 triggers an elastography measurement, here a transient elastography measurement. More precisely, he triggers the emission of a low frequency, transient elastic wave (for instance a shear wave), and of ultrasound shots transmitted to track how the subject's tissue is moved by this elastic wave. The elastography module 21 acquires and processes the signals received in response, to determine a value of the mechanical parameter mentioned above (e.g.: liver stiffness), in step S13. This transient elastography measurement could be repeated several times. Besides, in step S13, a CAP value is determined (either on the basis of ultrasound echo signals acquired during the transient elastography measurement and/or on the basis of other ultrasound echo signals, acquired for instance in the interval between two successive transient elastography measurements).

The value of the mechanical parameter, and the CAP value thus measured are then sent to the control and processing system 4, which acquires them in step S1.

Then, in step $S_T$, the control and processing system 4 test whether the value of the mechanical parameter fulfils a given criterion, or not.

Here, the control and processing system 4 determines that the value of the mechanical parameter (e.g.: liver stiffness), acquired in step S1, does not fulfil the criterion when this value passes a threshold value, that correspond to a limit between values for which liver does not suffer from health impairment in average, and values for which liver may suffer from health impairment.

When the mechanical parameter is the Young's modulus E, this threshold value may be in the range 6 - 7 kPa, for instance. Indeed, a value below 6 - 7 kPa for the Young's modulus indicates that the subject under examination is not likely to suffer from liver fibrosis, regardless of the concentrations of liver enzymes in the subject's blood.

When the criterion mentioned above is fulfilled, the control and processing system 4 execute S3'. In step S3', the control and processing system 4 determines the health condition of the liver of the subject, taking into account the value of the at least one mechanical parameter, regardless of a concentration of liver enzymes (namely AST, ALT or GGT) in the subject's blood. And when this criterion is fulfilled, no blood sample is collected from the subject, nor analyzed.

On the contrary, if the criterion mentioned above is not fulfilled, then, after step $S_T$, the control and processing system 4 launches a liver enzyme measurement process, in step S20. In particular, in step S20, the control and processing system 4 controls the operator interface 5 so that it transmits information specifying that a liver enzyme concentration measurement is recommended and/or prompting the operator 100 to collect a capillary blood sample from the subject and to launch the analysis of this blood sample.

Then, in step S200, the operator 100 collects a capillary blood sample from the subject, directly using the capillary tube 13 that is fixed to the blood-test disposable 10. Then, the operator introduces the blood-test disposable 10, loaded with this blood sample, in the blood-test reader 3.

In step S23, the blood-test reader 3 determines the concentration of at least one liver enzyme in this blood sample. Here, the blood-test reader 3 determines more particularly the concentration of AST and ALT, using the colorimetric detection method presented above, implemented in the blood-test disposable 10.

Then, the blood-test disposable 3 transmits the value or values of liver enzyme concentration measured in step S23 to the control and processing system 4, which acquires it, or them, in step S2.

Then, in step S3, the control and processing system 4 determines the health condition of the subject, taking into account at least the value of the mechanical parameter acquired in step S1, and at least one of the values of liver enzyme concentration acquired in step S2. Then, the control and processing system 4 outputs data representative of the health condition thus determined. These data may be transmitted to the operator 100 by the operator interface 5, for instance. They may also be sent to a storage device or system, like a personal electronic health card (for storing these data in this microcircuit card), or like a delocalized/distributed health data storage system.

In the method of figure 11, the operation of the elastography device 2, and the operation of the blood-test device 3 are clearly coordinated with each other (as the liver enzyme measurement is launched after the mechanical parameter measurement, depending on the mechanical parameter value). And the operations of the elastography device 2 and of the blood-test device 3 are concomitant, that is to say that they occur during a given, limited time frame.

More specifically, it is clear that in the method of figure 11, a start-up time lag $T_S$ between the beginning of step S10 and the beginning of step S20 is limited (due to the way the control and processing system is programmed). In practice, the main part of this duration corresponds to the measurement step S100, during which the operator executes the manipulations required to probe the stiffness of the liver of the subject (probe positioning, measurement triggering, possible repetitions of the elastography measurement). In practice, $T_S$ is typically between 2 and 5 minutes, and generally smaller than 10 minutes (or at least smaller than 20 minutes).

Besides, in the method of figure 11, a time lag Tm between the measurement moment of the mechanical parameter (in step S100) and a measurement moment of the concentration of liver enzymes (at the end of step S23) is also limited. In practice, the main part of this duration corresponds to :

- the manipulations carried on by the operator 100 (capillary blood collection, insertion of the blood-test disposable 10 in the blood-test reader, which typically takes 1 - 3 minutes) and
- the time required for the completion of detection chemical reactions, which is usually smaller than 5 minutes (or at least smaller than 10 minutes), here, thanks to the specific features of the blood-test disposable 10 presented above.

So, in practice, Tm is typically between 6 and 15 minutes (and generally smaller than 30 minutes).

The total time $T_T$ required to execute the entire method of figure 11 is thus typically between 7 and 30 minutes, most often between 10 and 20 minutes (and anyhow smaller than an hour).

The health condition determination, carried on in step S3, is now presented in more detail.

In an embodiment outside the scope of the appended method claims, the health condition determined in step S3 may take the form of information specifying whether the liver of the subject is likely to be healthy or, on the contrary, likely to be compromised by a given disease like Fibrosis or Steatosis or by an inflammation.

In another embodiment outside the scope of the appended method claims, the health condition determined in step S3 may also specify more gradually whether the liver of the subject is likely to be healthy or not, for instance in the form of a disease stage. For example, this disease stage could be the widely used Fibrosis stage F0 to F4 (F0: no fibrosis; F1: minimal fibrosis; F2: moderate fibrosis; F3: severe fibrosis; F4: most severe fibrosis / cirrhosis).

The health condition determined in step S3 may concern fibrosis, but also steatosis (for which the CAP value turns out to be very useful) and/or liver inflammation (the values of concentration of liver enzymes providing very useful information regarding this point).

In an embodiment outside the scope of the appended method claims, in step S3, the health condition of the liver of the subject may be determined by:

- computing a value of a benchmark parameter (a "score") that depends at least on the mechanical parameter and on the concentration of the liver enzyme mentioned above, then
- comparing the value of the benchmark parameter thus obtained with one or more threshold values (for instance with a negative predictive value, and with a positive predictive value), and
- determining the health condition of the liver of the subject from the result of the comparison or comparisons in question.

Different disease stages could be associated respectively to different value ranges of the benchmark parameter mentioned above.

In accordance with method claim 14, the health condition determined in step S3 takes the form of the value of a benchmark parameter, that depends at least on the mechanical parameter and on the concentration of the liver enzyme mentioned above, and that is representative of the fact that the liver of the subject is more or less healthy.

In other words, this health condition could be an intermediate benchmark parameter value, useful to a health care professional to make a diagnosis regarding the liver of the subject, instead of corresponding to the final diagnosis itself.

The benchmark parameter mentioned above could be the "FAST" parameter presented above, that is computed according to formula F1 above, for example. As described above, this benchmark parameter depends on the liver stiffness LSM (namely the Young's modulus E), the CAP, and the concentration of AST in the subject's blood.

The benchmark parameter, or, more generally the health condition of the liver of the subject could be determined taking also into account the concentration of ALT in the subject's blood. So, the benchmark parameter in question could depend on the liver stiffness LSM, optionally the CAP, the concentration of AST in the subject's blood, and the concentration of ALT in the subject's blood.

And other characteristics of the subject's blood, such as the concentration of platelets, could also be considered, when determining the health condition of the liver of subject.

Other clinical parameters relative to the subject, such as age and gender can also be taken into account when computing the benchmark parameter (score) mentioned above (the score computation formulae may include such additional clinical parameters).

For example, the health condition of the liver of the subject could be determined taking into account: the concentration of AST, the concentration of ALT and the concentration of platelets in the subject's blood, the age of the subject and the liver stiffness LSM.

To this end, in an embodiment, the control and processing system 4 may:

- compute a value of the "FIB-4" benchmark parameter, and then

- determine the health condition of the subject on the basis of this FIB-4 value and of an LSM value acquired in step S1.

The FIB-4 benchmark parameter is defined in the abstract of the following article: "Development of a simple noninvasive index to predict significant fibrosis patients with HIV/HCV co-infection", by Sterling R. K. et al., Hepatology 2006, vol. 43, pp 1317-1325.

In such an embodiment, the FIB-4 value may be employed, for instance to confirm or on the contrary invalidate a health condition that was first identified on the basis of the LSM value.

The FIB-4 value may also be employed to refine a preliminary health condition identification based on the LSM value, helping in particular to decide between one health condition and another in cases for which the LSM value falls in an LSM "grey range", between a negative predictive value and a positive predictive value.

The FIB-4 value may also be taken into account by computing a composite score, as a function of both the FIB-4 value and the LSM value.

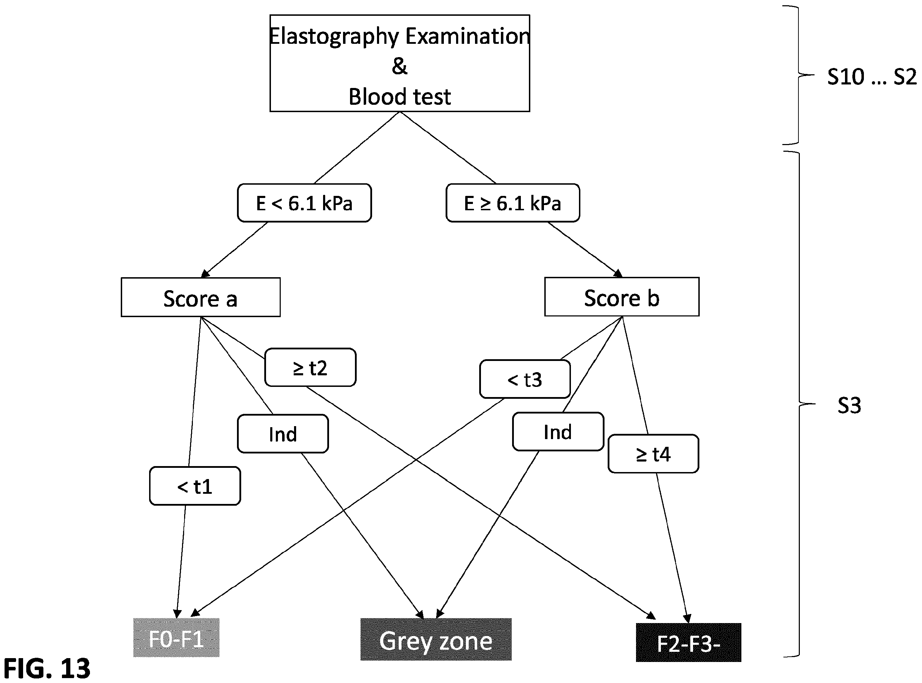

In step S3, different benchmark parameters (different scores) could be involved in the determination of the health condition of the subject, as represented in figure 13. This is beneficial, as a given benchmark parameter may be more appropriate than another to determine the health condition of the liver of the subject, depending on the health condition to be identified.

For instance, if benchmark parameters based on logistic regressions are employed, each of these benchmark parameters enables to make a binary decision. For example, a first benchmark parameter may allow for deciding if the subject's liver is compromised by F4 fibrosis or not, while a second benchmark parameter allow for deciding if the subject's fibrosis stage is F0-F1 or above. A finer, or more complete determination of the health condition of the liver of the subject could thus be achieved using a benchmark parameter that is selected, among different benchmark parameters, depending on the health condition to be identified. As the health condition of the subject is not known in advance, the selection of the benchmark parameter that is the more appropriate could be based on the preliminary result consisting of the value of the mechanical parameter (e.g.: liver stiffness) and/or ultrasound attenuation parameter acquired in step S1.

To this end, in some embodiments:

- different computation formulas are associated, in the memory of the control and processing system 4, to different ranges of values of the at least one mechanical parameter, each computation formula corresponding to a given benchmark parameter for liver health assessment and each computation formula enabling to compute the corresponding benchmark parameter from the at least one mechanical parameter and from the concentration of the at least one liver enzyme in the blood sample,

- the control and processing system 4 is programmed to select one of these benchmark parameters, by comparing the value of the at least one mechanical parameter, previously measured on the subject's liver, to the ranges of values associated respectively to these different benchmark parameters, and

- the control and processing system 4 is programmed to compute a value of the benchmark parameter previously selected, according to the formula associated to this benchmark parameter, in step S3.

This way to determine the health condition of the subject is illustrated, by way of example, in figure 13.

In this example, when the Young's modulus E of the subject's liver is below a given threshold, equal here to 6.1 kPa, a first benchmark parameter, referred to as score a, is selected. The value of score a is then computed, taking into account the value of E and the values of the concentration of AST and ALT in the subject's blood. When the value of score a is below a first threshold t1, the control and processing system 4 determines that the fibrosis stage of the liver of the subject is F0 or F1. And when the value of score a is above a second threshold t2, the control and processing system 4 determines that the fibrosis stage of the liver of the subject is F2, F3 or F4 (comprised between F2 and F4). When the value of score a falls between t1 and t2 ("grey zone"), the control and processing system 4 output data specifying that the health condition of the liver of the subject could not be determined.

On the contrary, when the Young's modulus E of the subject's liver is above the threshold mentioned above, equal to 6.1 kPa, a second benchmark parameter, referred to as score b, is selected. The value of score b is then computed, taking into account the value of E and the values of the concentration of AST and ALT in the subject's blood. When the value of score b is below a third threshold t3, the control and processing system 4 determines that the fibrosis stage of the liver of the subject is F0 or F1. And when the value of score b is above a fourth threshold t4, the control and processing system 4 determines that the fibrosis stage of the liver of the subject is F2, F3 or F4. And when the value of score b falls between t3 and t4 ("grey zone"), the control and processing system 4 output data specifying that the health condition of the liver of the subject could not be determined.

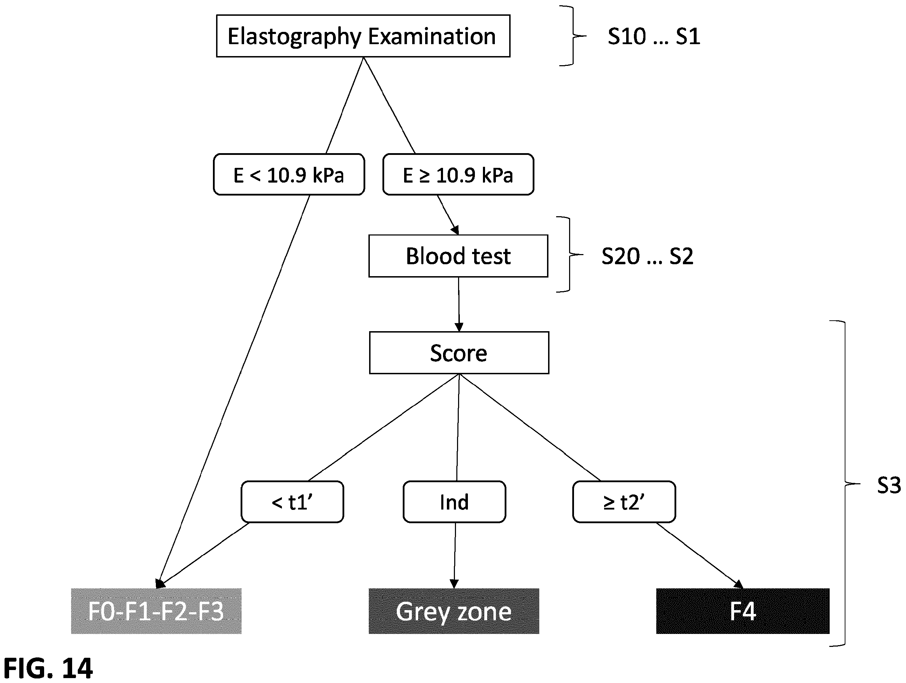

Figure 14 shows a calculation and test sequence executed in a particular example of implementation of the method in Figure 11. In this example, the method for determining the health condition of the liver of the subject is more particularly intended for determining whether the liver fibrosis stage of the subject is F4, or whether it is below (F0 to F3). So, the threshold value of E involved in step $S_T$ is relatively high, equal for instance to 10.9 kPa.

When E is below 10.9 kPa, after step $S_T$, the control and processing system 4 thus executes step S3', in which it determines directly, with no further calculation (in this particular example), that the liver fibrosis stage of the subject is comprised between F0 and F3 (equal to F0, F1, F2 or F3).

On the contrary, if E is above 10.9 kPa, then, after step $S_T$, the control and processing system 4 launches a liver enzyme measurement (steps S20 to S2). And then, in step S3, it computes a score value taking into account E and the values of concentration of AST and ALT. When the score value is below threshold t1', the control and processing system 4 determines that the liver fibrosis stage of the subject is comprised between F0 and F3. When the score value is above threshold t2', the control and processing system 4 determines that the liver fibrosis stage of the subject is F4. Otherwise, the control and processing system 4 outputs data specifying that the health condition of the liver of the subject could not be determined ("grey zone").

Figure 15 represents schematically a second embodiment of the method for determining a health condition of the liver of a subject, in accordance with the disclosed technology.

In this figure, the method is represented in the form of a flowchart, with similar conventions as in figure 11 (in particular, the direction in which the time t is elapsed corresponds to the vertical downward direction).

In this second embodiment, the liver enzyme measurement process and the mechanical parameter measurement process are launched almost in parallel, with a short delay $T_S'$ between their respective start. The liver enzyme measurement process is launched first (in step S20'). Then, once a blood sample has been collected from the subject (in step S200), and once the blood-test disposable 10 has been introduced in the reader 3 (at the beginning of step S23), the mechanical parameter measurement process is launched without delay (in step S10).

So, the time required for the detection chemical reactions to occur, which is typically between 5 and 10 minutes, is employed to measure, in parallel, the mechanical parameter mentioned above. This allows for reducing the total time $T_T'$ required to determine the health condition of the liver of the subject.

So, the method of figure 15 starts with step S20', during which the control and processing system 4 launches a liver enzyme measurement process. In particular, in step S20', the control and processing system 4 controls the operator interface 5 so that it transmits information prompting the operator 100 to collect a capillary blood sample from the subject and to launch the analysis of this blood sample. In step S22, the operator interface 5 transmits this information to the operator 100, here by displaying it. Then, in step S200, the operator collects a capillary blood sample from the subject and introduce the blood-test disposable 10, loaded with this blood sample, in the blood-test reader 3 (just like in the method of figure 11). The step S23 of analyzing the blood sample then starts. At the beginning of step S23, just after the blood-test disposable 10 was inserted in the blood-test reader 3, the blood-test reader 3 transmits to the control and processing system 4 information specifying that the blood-test disposable 10 has been inserted in the blood-test reader 3. This information is received by the control and processing system 4 in step S24. Once this information, confirming that the blood sample analysis has started, has been received, the control and processing system 4 launches the mechanical parameter measurement process, in step S10. Here, this measurement process comprises the steps S10, S11, S12, S100, S13 and then S1, that have already been described, in reference to figure 11.

During the execution of steps S10 to S1, the chemical analysis of the blood sample continues, and leads finally to the determination of the respective values of the concentration of AST and of the concentration of ALT in the subject's blood, at the end of step S23. These values are then transmitted to the control and processing system 4, which acquires then in step S2. Then step S3 is executed, as described above in reference to figure 11.

In the method of figure 12, time Ts' is typically between 1 and 5 minutes. And the overall time Tm' between the beginning of the liver enzyme measurement (that is, step S200), and the end of all the measurements, either serologic or mechanical (which corresponds here to the end of step S23, or to the end of step S13, depending on which is the latest), is typically of 4 to 10 minutes. And the total time $T_T'$ required to execute the method, which is smaller than the total time $T_T$ required to execute the method of figure 11, is typically between 5 and 20 minutes, most often between 7 and 15 minutes (and anyhow, smaller than an hour).

## Claims

1.  A system (1; 1'; 1") for determining a health condition of the liver of a subject, the system comprising:

    - an elastography device (2; 2'; 2") configured to measure at least one mechanical parameter relative to the propagation of shear waves in liver,
    - a blood-test reader (3) and a blood-test disposable (10) associated to the blood test reader (3), the blood-test disposable being configured to receive a capillary blood sample and to be inserted in the blood test reader (3) and the blood-test reader being configured to determine a concentration of at least one liver enzyme in said blood sample, wherein:

- the blood-test disposable (10) comprises:

    - a capillary tube (13) for collecting said blood sample from said finger, and
    - reagents, appropriate to detect said at least one liver enzyme in said blood sample, and wherein

- the blood-test reader (3) is operatively connected to the elastography device (2 ;2' ;2"), and

- a control and processing system (4; 4'; 4"), comprising at least a processor and a memory, configured to control the elastography (2 ;2' ;2") device and the blood test reader (3), the control and processing system (4; 4'; 4") being programmed to execute the following steps:

    - S1 - acquiring a value of said at least one mechanical parameter, measured by the elastography device (2 ;2' ;2"),
    - S2 - acquiring a value of a concentration of said at least one liver enzyme in a blood sample collected from the subject, measured by the blood-test reader (3), and
    - S3 - determining the health condition of the liver of the subject, taking into account both the value of said at least one mechanical parameter and the value of the concentration of said at least one liver enzyme, said health condition being represented by a health or disease stage identified among different stages (F1, F2, F3, F4) of a given health condition classification, or being represented by a value of a benchmark parameter taking into account both the value of said at least one mechanical parameter and the value of the concentration of said at least one liver enzyme, or being represented both by said health or disease stage and by said value of said benchmark parameter, and outputting data representative of said health condition,

wherein the control and processing system (4; 4'; 4") is programmed:

A) to control the elastography device (2 ;2' ;2") and the blood test reader (3) so that they start respectively an elastography measurement process, and a blood test process including collection and analysis of said blood sample, within a single time frame, or
B) to execute the following steps:

    - S10 - controlling the elastography device (2 ;2' ;2") so that the elastography device starts an elastography measurement process, then
    - said step S1, then
    - if the value of said at least one mechanical parameter acquired in step S1 fulfils a given criterion:

        - S3' - determining the health condition of the liver of the subject taking into account the value of said at least one mechanical parameter regardless of a concentration of said at least one liver enzyme in the subject's blood, and outputting data representative of said health condition, while

    - if the value of said at least one mechanical parameter does not fulfil said criterion:

        - S20 - controlling an operator interface (5; 5') so that said interface transmits information specifying that a blood test is recommended for the liver health assessment of the subject and/or prompting an operator (100) to collect a capillary blood sample from the subject and to launch the analysis of this blood sample, then

            - said step S2, and then
            - said step S3, or

C) to execute the following steps:

    - S20'- controlling an operator interface (5; 5') so that said interface transmits information prompting an operator (100) to collect a capillary blood sample from the subject and to launch the analysis of this blood sample, then, once the blood-test disposable (10) has been inserted in the blood-test reader (3),
    - S10 - controlling the elastography device (2 ;2' ;2") so that the elastography device starts an elastography measurement process, then
- said steps S1 and S2, and then,
- said step S3.

2. The system (1; 1'; 1") of claim 1, comprising the features of alternative A), wherein the control and processing system (4; 4'; 4") is programmed so that said time frame has a duration (Ts; Ts') of 30 minutes at most.

3. The system (1; 1'; 1") of claim 1 or 2, wherein the blood-test disposable (10) is configured so that said blood sample, collected by means of said capillary tube (13), has a volume of 60 microliters at most.

4. The system (1; 1'; 1") of claim 1, comprising the features of alternative B), wherein the control and processing system (4; 4'; 4") is programmed to determine that the value of said at least one mechanical parameter does not fulfil said criterion when said value passes a threshold value corresponding to a limit between values for which liver does not suffer from health impairment in average, and values for which liver may suffer from health impairment.

5. The system (1; 1'; 1") of any of claims 1 to 4, wherein:

- different computation formulas are associated, in the memory of the control and processing system (4; 4'; 4"), to different ranges of values of said at least one mechanical parameter, each computation formula corresponding to a given benchmark parameter for liver health assessment and each computation formula enabling to compute the corresponding benchmark parameter from said at least one mechanical parameter and from the concentration of said at least one liver enzyme in said blood sample,
- the control and processing system (4; 4'; 4") is programmed to select one of these benchmark parameters, by comparing the value of said at least one mechanical parameter, previously measured on the subject's liver, to the ranges of values associated respectively to these different benchmark parameters, and wherein
- the control and processing system (4; 4'; 4") is programmed to compute a value of the benchmark parameter previously selected, according to the formula associated to this benchmark parameter, in step S3.

6. The system (1; 1'; 1") of any of claims 1 to 5, wherein the capillary tube (13) is fixed to a part (12) of said disposable (10).

7. The system (1; 1'; 1") of claim 6, wherein the blood test disposable (10) comprises:

- a cartridge (11), containing said reagents, and
- a detachable plug (12),

the capillary tube (13) being fixed to the detachable plug (12), or to the cartridge, the plug (12) and the cartridge (11) being configured so that:

- the plug (12) can be detached from the cartridge (11) and then re-plugged onto the cartridge, and so that
- when the plug (12) is plugged onto the cartridge (11), the capillary tube (13) is hosted inside the disposable (10) and the blood sample collected by the capillary tube comes into contact with said reagents.

8. The system (1; 1'; 1") of any of claims 1 to 7, wherein

said at least one liver enzyme is one of: aspartate aminotransferase, hereinafter "AST", alanine aminotransferase, hereinafter "ALT", gamma-glutamyl transferase, hereinafter "GGT",
said reagents are appropriate to detect said at least one liver enzyme optically,
the blood-test disposable (10) is configured so that at least a part of the blood sample mixes with said reagents in a first reaction zone (Z1),
the blood-test reader (3) comprises at least a light source and a light sensor for determining the concentration of said at least one liver enzyme in said blood sample, by means of a light reflectance and/or transmittance measurement at the first reaction zone (Z1).

9. The system (1; 1'; 1") of any of claims 1 to 8, wherein the blood-test disposable (10) comprises a filtering membrane (110) arranged to filter blood red cells out of the blood sample collected from the subject in order to obtain a plasma sample, and is configured to bring at least a part of said plasma sample into contact with said reagents.

10. The system (1; 1'; 1") of any of claims 1 to 9, wherein:

- said at least one liver enzyme is AST or ALT,
- said reagents comprise alpha-Ketoglutarate, and L-Aspartic Acid or L-alanine respectively,
- the blood-test disposable (10) comprises the following catalysts: pyruvate oxidase and peroxidase, and comprises an indicator, that becomes colored when reacting with a product of the set of reactions that occur when said at least one liver enzyme is mixed with said reagents.

11. The system (1; 1'; 1") of claim 10, wherein:

- the blood-test disposable (10) comprises a liquid and an onboard control (119) of activity of said catalysts, said onboard control (119) comprising:

- a dry control substrate (116) containing oxaloacetate, and
- a dry control pad (117) containing said catalysts and said indicator, distinct from the control substrate (116),

the blood-test disposable (10) being configured so that the liquid soaks the control substrate (116) and the control pad (117) when the blood-test disposable (10) is inserted in the blood-test reader (3) or when said blood sample is received in the blood-test disposable (10).

12. The system (1; 1'; 1") of claim 11, wherein said liquid is a liquid buffer contained in a breakable blister (114) configured to break when the blood-test disposable (10) is inserted into the blood-test reader (3).

13. The system (1; 1'; 1") of any of claims 1 to 12, further configured to allow for determining a platelets count in said blood sample or in another blood sample collected from the subject, and wherein the control and processing system (4; 4'; 4") is programmed in order to determine said health condition, in step S3, taking also into account said platelets count.

14. A method for determining a health condition of the liver of a subject, the health condition being in form of a value of a benchmark parameter, by means of a system (1; 1'; 1") comprising:

- an elastography device (2; 2'; 2") configured to measure at least one mechanical parameter relative to the propagation of shear waves in liver,
- a blood-test reader (3) and a blood-test disposable (10) associated to the blood test reader (3), the blood-test disposable being configured to receive a capillary blood sample and to be inserted in the blood test reader (3), the blood-test reader being configured to determine a concentration of at least one liver enzyme in said blood sample, wherein:

- the blood-test disposable (10) comprises:

- a capillary tube (13) for collecting said blood sample from said finger, and
- reagents, appropriate to detect said at least one liver enzyme in said blood sample, and wherein

- the blood-test reader (3) is operatively connected to the elastography device (2; 2'; 2"), and

- a control and processing system (4; 4'; 4"), comprising at least a processor and a memory, configured to control the elastography device (2; 2'; 2") and the blood test reader (3),

the method comprising the following steps:

- S100 - measuring a value of said mechanical parameter using the elastography device (2; 2'; 2"), for the liver of the subject under examination,
- S1 - acquiring by the control and processing system (4; 4'; 4") the value of said at least one mechanical parameter, measured by the elastography device,
- S200 - collecting a capillary blood sample from the subject by means of said capillary tube (13), and then introducing the blood-test disposable (10) in the blood-test reader (3),
- S2 - acquiring by the control and processing system (4; 4'; 4") a value of a concentration of said at least one liver enzyme in a blood sample collected from the subject, measured by the blood test reader (3), and
- S3 - determining by the control and processing system the of the benchmark parameter taking into account both the value of said at least one mechanical parameter and the value of the concentration of said at least one liver enzyme, and outputting data representative of said value of the benchmark parameter, wherein

A) steps S100 and S200 are executed within a single time frame having a maximum, preset duration (Tm; Tm'), or wherein

B) steps S100 and S1 are executed first, and wherein the method comprises then the following steps:

- if the value of said at least one mechanical parameter acquired in step S1 fulfils a given criterion:

- S3' - the control and processing system (4; 4'; 4") determines the value of the benchmark parameter taking into account the value of said at least one mechanical parameter regardless of a concentration of said at least one liver enzyme in the subject's blood, and outputs data representative of said value of the benchmark parameter, while

- if the value of said at least one mechanical parameter does not fulfil said criterion:

- S20 - the control and processing system (4; 4'; 4") controls an operator interface (5; 5') so that said interface transmits information specifying that a liver enzyme concentration measurement is recommended for the liver health assessment of the subject and/or prompting an operator (100) to collect a capillary blood sample from the subject and to launch the analysis of this blood sample, then
- said step S200 and said step S2, and then
- said step S3, or wherein

C) the following steps are executed:

- S20'- controlling an operator interface (5; 5') so that said interface transmits information prompting an operator (100) to collect the capillary blood sample from the subject and to launch the analysis of this blood sample, then, once the blood-test disposable (10) has been inserted in the blood-test reader (3),
- S10 - controlling the elastography device (2 ;2' ;2") so that the elastography device starts an elastography measurement process, then
- said steps S1 and S2, and then,
- said step S3.

15. The method of claim 14, comprising the steps of alternative A), wherein the control and processing system outputs an error message when steps S100 and S200 are not executed within a single time frame having a maximum, preset duration (Tm; Tm').

16. The method of claim 14, comprising the steps of alternative B), wherein the control and processing system (4; 4'; 4") determines that the value of said at least one mechanical parameter does not fulfil said criterion when said value passes a threshold value corresponding to a limit between values for which liver does not suffer from health impairment in average, and values for which liver may suffer from health impairment.

**Patentansprüche**

1. System (1; 1'; 1") zum Bestimmen eines Gesundheitszustands der Leber einer Person, wobei das System umfasst:

- eine Elastographievorrichtung (2; 2'; 2"), die so ausgelegt ist, dass sie mindestens einen mechanischen Parameter misst, der sich auf die Ausbreitung von Scherwellen in der Leber bezieht,
- ein Bluttest-Lesegerät (3) und eine Einweg-Bluttestvorrichtung (10), die mit dem Bluttest-Lesegerät (3) verbunden ist, wobei die Einweg-Bluttestvorrichtung so ausgelegt ist, dass sie eine Kapillarblutprobe aufnimmt und in das Bluttest-Lesegerät (3) einführt, und das Bluttest-Lesegerät so ausgelegt ist, dass es eine Konzentration von mindestens einem Leberenzym in der Blutprobe bestimmt, wobei:

- die Einweg-Bluttestvorrichtung (10) umfasst:

- ein Kapillarröhrchen (13) zur Entnahme der Blutprobe aus dem Finger, und
- Reagenzien, die sich zum Nachweis des mindestens einen Leberenzyms in der Blutprobe eignen,

und in dem das Bluttest-Lesegerät (3) mit der Elastographievorrichtung (2; 2'; 2") wirkverbunden ist, und

- ein Steuer- und Verarbeitungssystem (4; 4'; 4"), das mindestens einen Prozessor und einen Speicher umfasst und so ausgelegt ist, dass es die Elastographievorrichtung (2; 2'; 2") und das Bluttest-Lesegerät (3) steuert, wobei das Steuer- und Verarbeitungssystem (4; 4'; 4") so programmiert ist, dass es die folgenden Schritte ausführt:

- S1 - Erfassen eines Wertes des mindestens einen mechanischen Parameters, der von der Elastographievorrichtung (2; 2'; 2") gemessen wird,
- S2 - Erfassen eines Wertes einer Konzentration des mindestens einen Leberenzyms in einer von der Person entnommenen Blutprobe, gemessen mit dem Bluttest-Lesegerät (3), und
- S3 - Bestimmen des Gesundheitszustands der Leber der Person, wobei sowohl der Wert des mindestens einen mechanischen Parameters als auch der Wert der Konzentration des mindestens einen Leberenzyms berücksichtigt werden, wobei der Gesundheitszustand durch einen Gesundheits- oder Krankheitsgrad dargestellt wird, der aus verschiedenen Graden (F1, F2, F3, F4) einer gegebenen Gesundheitszustandsklassifikation identifiziert wird, oder durch einen Wert eines Referenzparameters dargestellt wird, der sowohl den Wert des mindestens einen mechanischen Parameters als auch den Wert der Konzentration des mindestens einen Leberenzyms berücksichtigt, oder sowohl durch den Gesundheits- oder Krankheitsgrad als auch durch den Wert des Referenzparameters dargestellt wird, und Daten liefert, die für den Gesundheitszustand repräsentativ sind,

wobei das Steuer- und Verarbeitungssystem (4; 4'; 4") programmiert ist:

A) - um die Elastographievorrichtung (2; 2'; 2") und das Bluttest-Lesegerät (3) so zu steuern, dass sie jeweils einen ElastographieMessprozess und einen Bluttestprozess, der die Entnahme und Analyse der Blutprobe umfasst, innerhalb eines einzelnen Zeitabschnitts starten, oder
B) - um die folgenden Schritte auszuführen:

- S10 - Steuern der Elastographievorrichtung (2; 2'; 2"), so dass die Elastographievorrichtung einen Elastographiemessprozess startet, und dann
- den Schritt S1, und,
- wenn der Wert des mindestens einen mechanischen Parameters, der in Schritt S1 erfasst wurde, ein gegebenes Kriterium erfüllt:

- S3' - Bestimmen des Gesundheitszustands der Leber der Person unter Berücksichtigung des Werts des mindestens einen mechanischen Parameters unabhängig von einer Konzentration des mindestens einen Leberenzyms im Blut der Person und Bereitstellen von Daten, die für den Gesundheitszustand repräsentativ sind, während

- wenn der Wert des mindestens einen mechanischen Parameters das Kriterium nicht erfüllt:

- S20 - Steuern einer Bedienerschnittstelle (5; 5'), so dass die Schnittstelle eine Information überträgt, die angibt, dass ein Bluttest zur Beurteilung der Gesundheit der Leber der Person empfohlen wird, und/oder einen Bediener (100) auffordert, eine Kapillarblutprobe von der Person zu entnehmen und die Analyse dieser Blutprobe zu starten, und dann

- den Schritt S2, sowie
- den Schritt S3, oder

C) - um die folgenden Schritte auszuführen:

- S20'- Steuern einer Bedienerschnittstelle (5; 5'), so dass die Schnittstelle eine Information überträgt, die einen Bediener (100) auffordert, eine Kapillarblutprobe von der Person zu entnehmen und die Analyse dieser Blutprobe zu starten, und dann, nachdem die Einweg-Bluttestvorrichtung (10) in das Bluttest-Lesegerät (3) eingeführt worden ist,
- S10 - Steuern der Elastographievorrichtung (2; 2'; 2"), so dass die Elastographievorrichtung einen Elastographiemessprozess startet, und dann
- die Schritte S1 und S2, sowie
- den Schritt S3.

2. System (1; 1'; 1") nach Anspruch 1, mit den Besonderheiten der Variante A), in dem das Steuer- und Verarbeitungssystem (4; 4'; 4") so programmiert ist, dass die Zeitspanne eine Dauer (Ts; Ts') von höchstens 30 Minuten hat.

3. System (1; 1'; 1") nach Anspruch 1 oder 2, in dem die Einweg-Bluttestvorrichtung (10) so ausgelegt ist, dass die Blutprobe, die mittels des Kapillarröhrchens (13) entnommen wird, ein Volumen von höchstens 60 Mikrolitern hat.

4. System (1; 1'; 1") nach Anspruch 1, mit den Besonderheiten der Variante B), in dem das Steuer- und Verarbeitungssystem (4; 4'; 4") so programmiert ist, dass es bestimmt, dass der Wert des mindestens einen mechanischen Parameters das Kriterium nicht erfüllt, wenn der Wert einen Grenzwert überschreitet, der einer Grenze zwischen Werten, bei denen die Leber normalerweise keine Gesundheitsschädigung erleidet, und Werten, bei denen die Leber eine Gesundheitsschädigung erleiden kann, entspricht.

5. System (1; 1';1") nach einem der Ansprüche 1 bis 4, in dem:

- verschiedene Berechnungsformeln im Speicher des Steuer- und Verarbeitungssystems (4; 4'; 4") mit verschiedenen Wertebereichen des mindestens einen mechanischen Parameters verknüpft sind, wobei jede Berechnungsformel einem gegebenen Referenzparameter zur Beurteilung der Lebergesundheit entspricht und jede Berechnungsformel die Berechnung des entsprechenden Referenzparameters aus dem mindestens einen mechanischen Parameter und der Konzentration des mindestens einen Leberenzyms in der Blutprobe ermöglicht,
- das Steuer- und Verarbeitungssystem (4; 4'; 4") so programmiert ist, dass es einen dieser Referenzparameter auswählt, indem es den Wert des mindestens einen mechanischen Parameters, der zuvor an der Leber der Person gemessen wurde, mit den Wertebereichen vergleicht, die jeweils diesen verschiedenen Referenzparametern zugeordnet sind, und bei dem
- das Steuer- und Verarbeitungssystem (4; 4'; 4") so programmiert ist, dass es in Schritt S3 einen Wert des zuvor ausgewählten Referenzparameters gemäß der mit diesem Referenzparameter verbundenen Formel berechnet.

6. System (1; 1'; 1") nach einem der Ansprüche 1 bis 5, in dem das Kapillarröhrchen (13) an einem Teil (12) der Einwegvorrichtung (10) befestigt ist.

7. System (1; 1'; 1") nach Anspruch 6, in dem die Einweg-Bluttestvorrichtung (10) umfasst:

- eine Patrone (11), die die Reagenzien enthält, und
- eine abnehmbare Kappe (12),

wobei das Kapillarrohr (13) an dem abnehmbaren Stopfen (12) oder an der Patrone befestigt ist und der Stopfen (12) und die Patrone (11) so ausgelegt sind, dass:

- der Stopfen (12) von der Patrone (11) abgenommen und dann wieder auf die Patrone aufgesetzt werden kann, um sie zu verschließen, und so dass
- wenn der Stopfen (12) auf die Patrone (11) gesetzt wird, um sie zu verschließen, das Kapillarröhrchen (13) im Inneren der Einwegvorrichtung (10) untergebracht ist und die durch das Kapillarröhrchen entnommene Blutprobe mit den Reagenzien in Kontakt kommt.

8. System (1; 1';1") nach einem der Ansprüche 1 bis 7, in dem das mindestens eine Leberenzym eines ist von: Aspartat-Aminotransferase, im Folgenden "AST", Alanin-Aminotransferase, im Folgenden "ALT" und Gamma-Glutamyl-Transferase, im Folgenden "GGT",

wobei die Reagenzien zum optischen Nachweis des mindestens einen Leberenzyms geeignet sind und die Einweg-Bluttestvorrichtung (10) so ausgelegt ist, dass sich mindestens ein Teil der Blutprobe mit den Reagenzien in einer ersten Reaktionszone (Z1) vermischt,
das Bluttest-Lesegerät (3) mindestens eine Lichtquelle und einen Lichtsensor umfasst, um die Konzentration des mindestens einen Leberenzyms in der Blutprobe mittels einer Messung des Reflexionsvermögens und/oder der Lichtdurchlässigkeit in der ersten Reaktionszone (Z1) zu bestimmen.

9. System (1; 1'; 1") nach einem der Ansprüche 1 bis 8, in dem die Einweg-Bluttestvorrichtung (10) eine Filtermembran (110) umfasst, die so angeordnet ist, dass sie rote Blutkörperchen aus der Blutprobe filtert, die von der Person entnommen wurde, um eine Plasmaprobe zu erhalten, und so ausgelegt ist, dass sie mindestens einen Teil der Plasmaprobe mit den Reagenzien in Kontakt bringt.

10. System (1; 1';1") nach einem der Ansprüche 1 bis 9, in dem:

- das mindestens eine Leberenzym AST oder ALT ist,
- die Reagenzien jeweils Alpha-Ketoglutarat und L-Asparaginsäure oder L-Alanin umfassen,
- die Einweg-Bluttestvorrichtung (10) die folgenden Katalysatoren umfasst: Pyruvatoxidase und Peroxidase, und einen Indikator umfasst, der sich färbt, wenn er mit einem Produkt aus der Reihe von Reaktionen reagiert, die auftreten, wenn das mindestens eine Leberenzym mit den Reagenzien gemischt wird.

11. System (1; 1'; 1") nach Anspruch 10, bei dem:

- die Einweg-Bluttestvorrichtung (10) eine Flüssigkeit und einen eingebetteten Indikator (119) für die Aktivität der Katalysatoren umfasst, wobei der eingebettete Indikator (119) umfasst:

  - ein trockenes Kontrollsubstrat (116), das Oxaloacetat enthält, und
  - ein trockenes Kontrollplättchen (117), das die Katalysatoren und den Indikator enthält und sich vom Kontrollsubstrat (116) unterscheidet,

wobei die Einweg-Bluttestvorrichtung (10) so ausgelegt ist, dass die Flüssigkeit das Kontrollsubstrat (116) und das Kontrollplättchen (117) durchtränkt, wenn die Einweg-Bluttestvorrichtung (10) in das Bluttest-Lesegerät (3) eingeführt wird oder wenn die Blutprobe in der Einweg-Bluttestvorrichtung (10) aufgenommen wird.

12. System (1; 1'; 1") nach Anspruch 11, in dem die Flüssigkeit ein flüssiger Puffer ist, der in einer zerbrechlichen Blisterpackung (114) enthalten ist, die so ausgelegt ist, dass sie zerbricht, wenn die Einweg-Bluttestvorrichtung (10) in das Bluttest-Lesegerät (3) eingeführt wird.

13. System (1; 1'; 1") nach einem der Ansprüche 1 bis 12, das außerdem so ausgelegt ist, dass es die Bestimmung einer Thrombozytenzahl in der Blutprobe oder in einer anderen Blutprobe, die der Person entnommen wurde, ermöglicht, und bei dem das Steuer- und Verarbeitungssystem (4; 4'; 4") so programmiert ist, dass es den Gesundheitszustand in Schritt S3 auch unter Berücksichtigung der Thrombozytenzahl bestimmt.

14. Verfahren zur Bestimmung eines Gesundheitszustands der Leber einer Person, wobei der Gesundheitszustand in Form eines Werts eines Referenzparameters vorliegt, mittels eines Systems (1; 1'; 1"), das Folgendes umfasst:

- eine Elastographievorrichtung (2; 2'; 2"), die so ausgelegt ist, dass sie mindestens einen mechanischen Parameter misst, der sich auf die Ausbreitung von Scherwellen in der Leber bezieht,
- ein Bluttest-Lesegerät (3) und eine Einweg-Bluttestvorrichtung (10), die mit dem Bluttest-Lesegerät (3) verbunden ist, wobei die Einweg-Bluttestvorrichtung so ausgelegt ist, dass sie eine Kapillarblutprobe aufnimmt und in das Bluttest-Lesegerät (3) einführt, wobei das Bluttest-Lesegerät so ausgelegt ist, dass es eine Konzentration von mindestens einem Leberenzym in der Blutprobe bestimmt, wobei:

  - die Einweg-Bluttestvorrichtung (10) umfasst:

    - ein Kapillarröhrchen (13) zur Entnahme der Blutprobe aus dem Finger, und
    - Reagenzien, die sich zum Nachweis des mindestens einen Leberenzyms in der Blutprobe eignen, und wobei

  - das Bluttest-Lesegerät (3) mit der Elastographievorrichtung (2; 2'; 2") wirkverbunden ist, und

- ein Steuer- und Verarbeitungssystem (4; 4'; 4"), das mindestens einen Prozessor und einen Speicher umfasst und so ausgelegt ist, dass es die Elastographievorrichtung (2; 2'; 2") und das Bluttest-Lesegerät (3) steuern kann,

wobei das Verfahren die folgenden Schritte umfasst:

- S100 - Messen eines Wertes des genannten mechanischen Parameters mithilfe der Elastographievorrichtung (2; 2'; 2") für die Leber der zu untersuchenden Person,
- S1 - Erfassen des Wertes des mindestens einen mechanischen Parameters, der von der Elastographievorrichtung gemessen wurde, durch das Steuer- und Verarbeitungssystem (4; 4'; 4"),
- S200 - Entnahme einer Kapillarblutprobe von der Person mithilfe des Kapillarröhrchens (13) und dann Einführen der Einweg-Bluttestvorrichtung (10) in das Bluttest-Lesegerät (3),

- S2 - Erfassen durch das Steuer- und Verarbeitungssystem (4; 4'; 4") eines Wertes einer Konzentration des mindestens einen Leberenzyms in der von der Person entnommenen Blutprobe, die durch das Bluttest-Lesegerät (3) gemessen wurde, und

- S3 - Bestimmen des Wertes des Referenzparameters durch das Steuer- und Verarbeitungssystem unter Berücksichtigung sowohl des Wertes des mindestens einen mechanischen Parameters als auch des Wertes der Konzentration des mindestens einen Leberenzyms, und Bereitstellen von Daten, die für den Wert des Referenzparameters repräsentativ sind, wobei

A) die Schritte S100 und S200 in einem einzelnen Zeitabschnitt mit einer vordefinierten maximalen Dauer (Tm; Tm') ausgeführt werden, oder wobei

B) die Schritte S100 und S1 zuerst ausgeführt werden, und wobei das Verfahren dann die folgenden Schritte umfasst:

- wenn der Wert des mindestens einen mechanischen Parameters, der in Schritt S1 erfasst wurde, ein gegebenes Kriterium erfüllt:

- S3' - das Steuer- und Verarbeitungssystem (4; 4'; 4") den Wert des Referenzparameters bestimmt, indem es den Wert des mindestens einen mechanischen Parameters unabhängig von einer Konzentration des mindestens einen Leberenzyms im Blut der Person berücksichtigt, und Daten bereitstellt, die für den Wert des Referenzparameters repräsentativ sind, während

- wenn der Wert des mindestens einen mechanischen Parameters das Kriterium nicht erfüllt:

- S20 - das Steuer- und Verarbeitungssystem (4; 4'; 4") eine Bedienerschnittstelle (5; 5') so steuert, dass die Schnittstelle eine Information überträgt, die angibt, dass eine Messung der Leberenzymkonzentration zur Beurteilung der Lebergesundheit der Person empfohlen wird, und/oder einen Bediener (100) auffordert, eine Kapillarblutprobe von der Person zu entnehmen und die Analyse dieser Blutprobe zu starten, und dann

- den Schritt S200 und den Schritt S2 sowie

- den Schritt S3, oder in dem

C) die folgenden Schritte ausgeführt werden:

- S20' - Steuern einer Bedienerschnittstelle (5; 5'), so dass diese Schnittstelle eine Information überträgt, die einen Bediener (100) auffordert, die Kapillarblutprobe von der Person zu entnehmen und die Analyse dieser Blutprobe zu starten, und dann, nachdem die Einweg-Bluttestvorrichtung (10) in das Bluttest-Lesegerät (3) eingeführt worden ist, die Analyse dieser Blutprobe zu starten,

- S10 - Steuern der Elastographievorrichtung (2; 2'; 2"), so dass die Elastographievorrichtung einen Elastographiemessprozess startet, und dann

- die Schritte S1 und S2, sowie

- den Schritt S3.

**15.** Verfahren nach Anspruch 14, die Schritte der Variante A) umfassend, in dem das Steuer- und Verarbeitungssystem eine Fehlermeldung ausgibt, wenn die Schritte S100 und S200 nicht innerhalb eines einzelnen Zeitintervalls mit einer vordefinierten maximalen Dauer (Tm; Tm') ausgeführt werden.

**16.** Verfahren nach Anspruch 14, die Schritte der Variante B) umfassend, in dem das Steuer- und Verarbeitungssystem (4; 4'; 4") bestimmt, dass der Wert des mindestens einen mechanischen Parameters das Kriterium nicht erfüllt, wenn der Wert einen Grenzwert überschreitet, der einer Grenze zwischen Werten, bei denen die Leber normalerweise keine Gesundheitsschädigung erfährt, und Werten, bei denen die Leber eine Gesundheitsschädigung erfahren kann, entspricht.

## Revendications

**1.** Système (1 ; 1' ; 1") permettant de déterminer un état de santé du foie d'un sujet, le système comprenant :

- un dispositif d'élastographie (2 ; 2' ; 2") configuré pour mesurer au moins un paramètre mécanique relatif à la propagation d'ondes de cisaillement dans le foie,
- un lecteur de test sanguin (3) et un dispositif jetable de test sanguin (10) associé au lecteur de test sanguin (3), le dispositif jetable de test sanguin étant configuré pour recevoir un échantillon de sang capillaire et pour être inséré dans le lecteur de test sanguin (3) et le lecteur de test sanguin étant configuré pour déterminer une concentration d'au moins une enzyme hépatique dans ledit échantillon de sang, dans lequel :

- le dispositif jetable de test sanguin (10) comprend :

- un tube capillaire (13) pour prélever ledit échantillon de sang sur ledit doigt, et
- des réactifs, convenables pour détecter ladite au moins une enzyme hépatique dans ledit échantillon de sang,

et dans lequel
- le lecteur de test sanguin (3) est relié opérationnellement au dispositif d'élastographie (2 ; 2' ; 2"), et

- un système de commande et de traitement (4 ; 4' ; 4"), comprenant au moins un processeur et une mémoire, configuré pour commander le dispositif d'élastographie (2 ; 2' ; 2") et le lecteur de test sanguin (3), le système de commande et de traitement (4 ; 4' ; 4") étant programmé pour exécuter les étapes suivantes :

- S1 - acquérir une valeur dudit au moins un paramètre mécanique, mesurée par le dispositif d'élastographie (2 ; 2' ; 2"),
- S2 - acquérir une valeur d'une concentration de ladite au moins une enzyme hépatique dans un échantillon de sang prélevé sur le sujet, mesurée par le lecteur de test sanguin (3), et
- S3 - déterminer l'état de santé du foie du sujet, en prenant en compte à la fois la valeur dudit au moins un paramètre mécanique et la valeur de la concentration de ladite au moins une enzyme hépatique, ledit état de santé étant représenté par un stade de santé ou de maladie identifié parmi différents stades (F1, F2, F3, F4) d'une classification d'état de santé donnée, ou étant représenté par une valeur d'un paramètre de référence prenant en compte à la fois la valeur dudit au moins un paramètre mécanique et la valeur de la concentration de ladite au moins une enzyme hépatique, ou étant représenté à la fois par ledit stade de santé ou de maladie et par ladite valeur dudit paramètre de référence, et fournir en sortie des données représentatives dudit état de santé,

dans lequel le système de commande et de traitement (4 ; 4' ; 4") est programmé :

A) - pour commander le dispositif d'élastographie (2 ; 2' ; 2") et le lecteur de test sanguin (3) de sorte qu'ils démarrent respectivement un processus de mesure d'élastographie, et un processus de test sanguin incluant un prélèvement et une analyse dudit échantillon de sang, dans un laps de temps unique, ou
B) - pour exécuter les étapes suivantes :

- S10 - commander le dispositif d'élastographie (2 ; 2' ; 2") de sorte que le dispositif d'élastographie démarre un processus de mesure d'élastographie, puis
- ladite étape S1, puis
- si la valeur dudit au moins un paramètre mécanique acquise à l'étape S1 remplit un critère donné :

- S3' - déterminer l'état de santé du foie du sujet en prenant en compte la valeur dudit au moins un paramètre mécanique indépendamment d'une concentration de ladite au moins une enzyme hépatique dans le sang du sujet, et fournir en sortie des données représentatives dudit état de santé, tandis que

- si la valeur dudit au moins un paramètre mécanique ne remplit pas ledit critère :

- S20 - commander une interface d'opérateur (5 ; 5') de sorte que ladite interface transmette une information spécifiant qu'un test sanguin est recommandé pour apprécier la santé du foie du sujet et/ou invitant un opérateur (100) à prélever un échantillon de sang capillaire sur le sujet et à lancer l'analyse de cet échantillon de sang, puis

- ladite étape S2, puis
- ladite étape S3, ou

C) - pour exécuter les étapes suivantes :

- S20' - commander une interface d'opérateur (5 ; 5') de sorte que ladite interface transmette une information invitant un opérateur (100) à prélever un échantillon de sang capillaire sur le sujet et à lancer l'analyse de cet échantillon de sang, puis, une fois que le dispositif jetable de test sanguin (10) a été inséré dans le lecteur de test sanguin (3),
- S10 - commander le dispositif d'élastographie (2 ; 2' ; 2") de sorte que le dispositif d'élastographie démarre un processus de mesure d'élastographie, puis
- lesdites étapes S1 et S2, puis,
- ladite étape S3.

2. Système (1 ; 1'; 1") selon la revendication 1, comprenant les caractéristiques de la variante A), dans lequel le système de commande et de traitement (4 ; 4' ; 4") est programmé de sorte que ledit laps de temps ait une durée (Ts ; Ts') de 30 minutes au plus.

3. Système (1 ; 1' ; 1") selon la revendication 1 ou 2, dans lequel le dispositif jetable de test sanguin (10) est configuré de sorte que ledit échantillon de sang, prélevé au moyen dudit tube capillaire (13), a un volume de 60 microlitres au plus.

4. Système (1 ; 1'; 1") selon la revendication 1, comprenant les caractéristiques de la variante B), dans lequel le système de commande et de traitement (4 ; 4' ; 4") est programmé pour déterminer que la valeur dudit au moins un paramètre mécanique ne remplit pas ledit critère lorsque ladite valeur franchit une valeur seuil correspondant à une limite entre des valeurs pour lesquelles le foie ne subit pas de détérioration de santé en moyenne, et des valeurs pour lesquelles le foie peut subir une détérioration de santé.

5. Système (1 ; 1' ; 1") selon l'une quelconque des revendications 1 à 4, dans lequel :

- différentes formules de calcul sont associées, dans la mémoire du système de commande et de traitement (4 ; 4' ; 4"), à différentes plages de valeurs dudit au moins un paramètre mécanique, chaque formule de calcul correspondant à un paramètre de référence donné pour l'appréciation de la santé du foie et chaque formule de calcul permettant de calculer le paramètre de référence correspondant à partir dudit au moins un paramètre mécanique et de la concentration de ladite au moins une enzyme hépatique dans ledit échantillon de sang,
- le système de commande et de traitement (4 ; 4' ; 4") est programmé pour sélectionner un de ces paramètres de référence, en comparant la valeur dudit au moins un paramètre mécanique, précédemment mesurée sur le foie du sujet, aux plages de valeurs associées respectivement à ces différents paramètres de référence, et dans lequel
- le système de commande et de traitement (4 ; 4' ; 4") est programmé pour calculer une valeur du paramètre de référence précédemment sélectionné, selon la formule associée à ce paramètre de référence, à l'étape S3.

6. Système (1 ; 1' ; 1") selon l'une quelconque des revendications 1 à 5, dans lequel le tube capillaire (13) est fixé à une partie (12) dudit dispositif jetable (10).

7. Système (1 ; 1' ; 1") selon la revendication 6, dans lequel le dispositif jetable de test sanguin (10) comprend :

- une cartouche (11), contenant lesdits réactifs, et
- un bouchon détachable (12),

le tube capillaire (13) étant fixé au bouchon détachable (12), ou à la cartouche, le bouchon (12) et la cartouche (11) étant configurés de sorte que :

- le bouchon (12) puisse être détaché de la cartouche (11) puis replacé sur la cartouche pour la boucher, et de sorte que
- lorsque le bouchon (12) est placé sur la cartouche (11) pour la boucher, le tube capillaire (13) est logé à l'intérieur du dispositif jetable (10) et l'échantillon de sang prélevé par le tube capillaire entre en contact avec lesdits réactifs.

8. Système (1 ; 1' ; 1") selon l'une quelconque des revendications 1 à 7, dans lequel

ladite au moins une enzyme hépatique est une parmi : aspartate aminotransférase, ci-après « AST », alanine aminotransférase, ci-après « ALT », gamma-glutamyl transférase, ci-après « GGT »,

lesdits réactifs sont convenables pour détecter ladite au moins une enzyme hépatique par voie optique,
le dispositif jetable de test sanguin (10) est configuré de sorte qu'au moins une partie de l'échantillon de sang se mélange avec lesdits réactifs dans une première zone de réaction (Z1),
le lecteur de test sanguin (3) comprend au moins une source de lumière et un capteur de lumière permettant de déterminer la concentration de ladite au moins une enzyme hépatique dans ledit échantillon de sang, au moyen d'une mesure de réflectance et/ou de transmittance de lumière au niveau de la première zone de réaction (Z1).

9. Système (1 ; 1' ; 1") selon l'une quelconque des revendications 1 à 8, dans lequel le dispositif jetable de test sanguin (10) comprend une membrane filtrante (110) agencée pour filtrer les globules rouges issus de l'échantillon de sang prélevé sur le sujet afin d'obtenir un échantillon de plasma, et est configuré pour mettre au moins une partie dudit échantillon de plasma en contact avec lesdits réactifs.

10. Système (1 ; 1' ; 1") selon l'une quelconque des revendications 1 à 9, dans lequel :

    - ladite au moins une enzyme hépatique est AST ou ALT,
    - lesdits réactifs comprennent respectivement de l'alpha-cétoglutarate et de l'acide L-aspartique ou de la L-alanine,
    - le dispositif jetable de test sanguin (10) comprend les catalyseurs suivants : pyruvate oxydase et peroxydase, et comprend un indicateur qui se colore lorsqu'il réagit avec un produit de l'ensemble de réactions qui se produisent lorsque ladite au moins une enzyme hépatique est mélangée avec lesdits réactifs.

11. Système (1 ; 1' ; 1") selon la revendication 10, dans lequel :

    - le dispositif jetable de test sanguin (10) comprend un liquide et un témoin embarqué (119) de l'activité desdits catalyseurs, ledit témoin embarqué (119) comprenant :

        - un substrat témoin sec (116) contenant de l'oxaloacétate, et
        - un plot témoin sec (117) contenant lesdits catalyseurs et ledit indicateur, distinct du substrat témoin (116),

    le dispositif jetable de test sanguin (10) étant configuré de sorte que le liquide imbibe le substrat témoin (116) et le plot témoin (117) lorsque le dispositif jetable de test sanguin (10) est inséré dans le lecteur de test sanguin (3) ou lorsque ledit échantillon de sang est reçu dans le dispositif jetable de test sanguin (10).

12. Système (1 ; 1' ; 1") selon la revendication 11, dans lequel ledit liquide est un tampon liquide contenu dans un emballage-coque cassable (114) configuré pour se casser lorsque le dispositif jetable de test sanguin (10) est inséré dans le lecteur de test sanguin (3).

13. Système (1 ; 1' ; 1") selon l'une quelconque des revendications 1 à 12, configuré en outre pour permettre de déterminer une numération plaquettaire dans ledit échantillon de sang ou dans un autre échantillon de sang prélevé sur le sujet, et dans lequel le système de commande et de traitement (4 ; 4' ; 4") est programmé pour déterminer ledit état de santé, à l'étape S3, en prenant également en compte ladite numération plaquettaire.

14. Procédé de détermination d'un état de santé du foie d'un sujet, l'état de santé se présentant sous la forme d'une valeur d'un paramètre de référence, au moyen d'un système (1 ; 1' ; 1") comprenant :

    - un dispositif d'élastographie (2 ; 2' ; 2") configuré pour mesurer au moins un paramètre mécanique relatif à la propagation d'ondes de cisaillement dans le foie,
    - un lecteur de test sanguin (3) et un dispositif jetable de test sanguin (10) associé au lecteur de test sanguin (3), le dispositif jetable de test sanguin étant configuré pour recevoir un échantillon de sang capillaire et pour être inséré dans le lecteur de test sanguin (3), le lecteur de test sanguin étant configuré pour déterminer une concentration d'au moins une enzyme hépatique dans ledit échantillon de sang, dans lequel :

        - le dispositif jetable de test sanguin (10) comprend :

            - un tube capillaire (13) pour prélever ledit échantillon de sang sur ledit doigt, et
            - des réactifs, convenables pour détecter ladite au moins une enzyme hépatique dans ledit échantillon de sang, et dans lequel

- le lecteur de test sanguin (3) est relié opérationnellement au dispositif d'élastographie (2 ; 2' ; 2"), et

- un système de commande et de traitement (4 ; 4' ; 4"), comprenant au moins un processeur et une mémoire, configuré pour commander le dispositif d'élastographie (2 ; 2' ; 2") et le lecteur de test sanguin (3),

le procédé comprenant les étapes suivantes :

- S100 - mesurer une valeur dudit paramètre mécanique à partir du dispositif d'élastographie (2 ; 2' ; 2"), pour le foie du sujet en examen,
- S1 - acquérir par le système de commande et de traitement (4 ; 4' ; 4") la valeur dudit au moins un paramètre mécanique, mesurée par le dispositif d'élastographie,
- S200 - prélever un échantillon de sang capillaire sur le sujet au moyen dudit tube capillaire (13), puis introduire le dispositif jetable de test sanguin (10) dans le lecteur de test sanguin (3),
- S2 - acquérir par le système de commande et de traitement (4 ; 4' ; 4") une valeur d'une concentration de ladite au moins une enzyme hépatique dans l'échantillon de sang prélevé sur le sujet, mesurée par le lecteur de test sanguin (3), et
- S3 - déterminer par le système de commande et de traitement la valeur du paramètre de référence en prenant en compte à la fois la valeur dudit au moins un paramètre mécanique et la valeur de la concentration de ladite au moins une enzyme hépatique, et fournir en sortir des données représentatives de ladite valeur du paramètre de référence, dans lequel

A) les étapes S100 et S200 sont exécutées dans un laps de temps unique ayant une durée maximale prédéfinie (Tm ; Tm'), ou dans lequel
B) les étapes S100 et S1 sont exécutées en premier, et dans lequel le procédé comprend alors les étapes suivantes :

- si la valeur dudit au moins un paramètre mécanique acquise à l'étape S1 remplit un critère donné :

- S3' - le système de commande et de traitement (4 ; 4' ; 4") détermine la valeur du paramètre de référence en prenant en compte la valeur dudit au moins un paramètre mécanique indépendamment d'une concentration de ladite au moins une enzyme hépatique dans le sang du sujet, et fournit en sortie des données représentatives de ladite valeur du paramètre de référence, tandis que

- si la valeur dudit au moins un paramètre mécanique ne remplit pas ledit critère :

- S20 - le système de commande et de traitement (4 ; 4' ; 4") commande une interface d'opérateur (5 ; 5') de sorte que ladite interface transmette une information spécifiant qu'une mesure de concentration d'enzymes hépatiques est recommandée pour apprécier la santé du foie du sujet et/ou invitant un opérateur (100) à prélever un échantillon de sang capillaire sur le sujet et à lancer l'analyse de cet échantillon de sang, puis
- ladite étape S200 et ladite étape S2, puis
- ladite étape S3, ou dans lequel

C) les étapes suivantes sont exécutées :

- S20' - commander une interface d'opérateur (5 ; 5') de sorte que cette interface transmette une information invitant un opérateur (100) à prélever l'échantillon de sang capillaire sur le sujet et à lancer l'analyse de cet échantillon de sang, puis, une fois que le dispositif jetable de test sanguin (10) a été inséré dans le lecteur de test sanguin (3),
- S10 - commander le dispositif d'élastographie (2 ; 2' ; 2") de sorte que le dispositif d'élastographie démarre un processus de mesure d'élastographie, puis
- lesdites étapes S1 et S2, puis,
- ladite étape S3.

**15.** Procédé selon la revendication 14, comprenant les étapes de la variante A), dans lequel le système de commande et de traitement fournit en sortie un message d'erreur lorsque les étapes S100 et S200 ne sont pas exécutées dans un laps de temps unique ayant une durée maximale prédéfinie (Tm ; Tm').

**16.** Procédé selon la revendication 14, comprenant les étapes de la variante B), dans lequel le système de commande et de traitement (4 ; 4' ; 4") détermine que la valeur dudit au moins un paramètre mécanique ne remplit pas ledit critère lorsque ladite valeur franchit une valeur seuil correspondant à une limite entre des valeurs pour lesquelles le foie ne subit pas de détérioration de santé en moyenne, et des valeurs pour lesquelles le foie peut subir une détérioration de santé.

EP 3 939 514 B1

**FIG. 1A**

FIG. 1B

FIG. 2

**FIG. 3**

**FIG. 4**

**FIG. 6**

FIG. 5

EP 3 939 514 B1

FIG. 7

FIG. 8

**AST**

L-Aspartic Acid + alpha-Ketoglutarate →(Aspartate Aminotransferase)→ Oxaloacetate + Glutamate  R1

Oxaloacetate →(Oxaloacetate Decarboxylase)→ Pyruvate + $CO_2$  R2

Pyruvate + Phosphate + $O_2$ + $H_2O$ →(Pyruvate Oxidase)→ Acetylphosphate + $CO_2$ + $H_2O_2$  R3

Indicator + $H_2O_2$ →(Peroxidase)→ Colored Dye + $H_2O$  R4

**ALT**

L-Alanine + alpha-Ketoglutarate →(Alanine Aminotransferase)→ Pyruvate + Glutamate  R5

Pyruvate + Phosphate + $O_2$ + $H_2O$ →(Pyruvate Oxidase)→ Acetylphosphate + $CO_2$ + $H_2O_2$  R3

Indicator + $H_2O_2$ →(Peroxidase)→ Colored Dye + $H_2O$  R4

**FIG. 9**

EP 3 939 514 B1

Input: collected blood sample (~30 µL)

110

Output: Plasma (~10-12 uL of plasma, not hemolysed)

Indication of Hemolysis – threshold (optical measurement without tempering the sample)

AST & ALT quantification

Controls of formulation main component (positive & negative controls using buffer + dried substrate)

Sample Collection

Filtering System

QC_H

ALT

AST

QC +

QC -

Substrate

Buffer (blister)

117

114

122

112

111

118

116

10

**FIG. 10**

FIG. 11

FIG. 12

FIG. 13

FIG. 14

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018140247 A

- WO 2015014763 A

**Non-patent literature cited in the description**

- **L. SANDRIN**. Transient elastography: a new non-invasive method for assessment of hepatic fibrosis. *Ultrasound in Medicine and Biology*, 2003, vol. 29 (12)
- **S. MULLER** ; **L. SANDRIN**. Liver stiffness: a novel parameter for the diagnosis of liver disease. *Hepatic Medicine: Evidence and Research*, 2010, vol. 2, 49-67

- **P. NEWSOME**. FibroScan-AST (FAST) score for the non-invasive identification of patients with non-alcoholic steatohepatitis with significant activity and fibrosis: a prospective derivation and global validation study. *The Lancet, Gastroenterology and Hepatology*, 01 April 2020, vol. 5 (4), 362-373
- **STERLING R. K. et al.** Development of a simple noninvasive index to predict significant fibrosis patients with HIV/HCV co-infection. *Hepatology*, 2006, vol. 43, 1317-1325